# EUROPEAN PATENT APPLICATION

(11) **EP 4 388 888 A1**
(43) Date of publication of application: **26.06.2024**
(21) Application number: 22383248.6
(22) Date of filing: 21.12.2022
(51) Int. Cl.: A23L 3/3571, A23C 19/11, C12N 1/06, C12N 15/09, C12Q 1/70, A61K 35/76, A61P 31/04

(54) **LISTERIA SPECIFIC BACTERIOPHAGE COMPOSITION**

(71) Applicant: Fundación AZTI - AZTI Fundazioa, 48160 Derio (Bizkaia) (ES)
(72) Inventor: Lasagabaster, Amaia, 48160 Bizkaia (ES); Jiménez, Elisa, 48160 Bizkaia (ES); Lavilla, María, 48160 Bizkaia (ES)
(74) Representative: Pons

(57) **Abstract**

The invention relates to a composition comprising a bacteriophage or comprising a combination of bacteriophages for preventing, treating or controlling contamination with, and/or growth of, *Listeria* spp. in a food product, on food processing equipments, or on food storage containers. The invention also discloses the use of said composition for the treatment of an infection of *Listeria* spp. and for the detection of *Listeria* spp.

## Description

The invention relates to the field of microbiology, specifically to a bacteriophage or a combination of bacteriophages, to a composition comprising said bacteriophage or a combination of bacteriophages for preventing, treating or controlling contamination with and/or growth of *Listeria* in a food product, on food processing equipment, or on food storage containers.

### BACKGROUND ART

*Listeria monocytogenes* is a well-known pathogen responsible for listeriosis, one of the most serious food- and feed-borne zoonotic diseases worldwide. Microscopically *L. monocytogenes* appears as a small gram-positive rod (0.5-2 µm × 0.5 µm), occurring singly or arranged in short chains, motile at 20-25 °C and non-sporeforming. It is aerobic and facultatively anaerobic, catalase-positive except for a few rare strains, oxidase negative and esculin positive. *Listeria* ferments many carbohydrates without producing gas. Strains of *L. monocytogenes* are D-xylose negative and produce lecithinase. They are generally β-haemolytic and L-rhamnose positive. *L. monocytogenes* is genetically diverse: The strains are classified into four evolutionary lineages (I-IV), 13 serotypes (1/2a, 1/2b, 1/2c, 3a, 3b, 3c, 4a, 4ab, 4b, 4c, 4d, 4e, and 7) based on conventional serotyping (somatic and flagellar antigens) and 4 major molecular serogroups (IIa., IIb, IIc and IVb) based on PCR tests.

Animal listeriosis is most commonly associated with encephalitis, abortion, septicaemia and mastitis in ruminants, but swine, horses, birds, rodents, fishes, crustaceans, and a wide range of animal species can also be affected. Listeriosis in wild and domestic animals is usually transmitted through the ingestion of contaminated feed and/or pet food, although it can also be transmitted through the upper respiratory tract mucosa, conjunctiva and wounds. In humans, listeriosis cases are generally asymptomatic but could also manifest as febrile gastroenteritis, meningitis, encephalitis, septicaemia or lead to preterm birth and spontaneous abortion. This pathogen can reach the food products by contaminated raw materials or by cross-contamination during different steps of food processing. The most common food vehicles implicated in foodborne outbreaks have been associated with fish products, meats, vegetables and soft cheeses.

In addition, the ability of *L. monocytogenes* to form biofilms on food/feed-contact surfaces, equipment, floors and drains, together with its high tolerance to disinfectants, are determining factors in the persistence of these bacteria, even for years, in production/processing facilities. Furthermore, *L. monocytogenes* is able to survive and grow at a wide range of temperature (0.4-45 _{°}C) and pH (4.7-9.2), high acidic solutions, high salt concentrations and under osmotic pressures. These exceptional capabilities make it very challenging to remove *L. monocytogenes* from production/processing facilities, equipment, and environments, therefore increasing the contamination risk of the final product. This is particularly critical for those food products covered by Regulation (EC) no 2073/2005 (European Union, 2005), that sets a food safety criterion for *L. monocytogenes.*

These data make *L. monocytogenes* of major concern for the public health sector and the entire food chain sector (primary production as well as food and feed industry) and underline the need to explore innovative approaches or weapons to combat these zoonotic pathogenic bacteria.

The use of bacteriophages as an innovative strategy against target pathogen is desirable since they are highly specific towards the bacteria of concern, they are harmless to plants, animals and humans, and they do not affect the existing commensal microbiota of the host, food, feed and/or the environment, or alter food/feed properties. Moreover, bacteriophages are self-replicating and self-limiting, meaning that they do only proliferate if there is a suitable host present.

However, there are also limitations to the use of bacteriophages as biocontrol agents and *Listeria* specific bacteriophages must fulfil certain requirements regarding their specificity, efficacy, stability and safety (Kakasis, A. and Panitsa, G., 2019. Int J Antimicrob Agents, 53(1): 16-21).

According to their life cycle, bacteriophages have been classified into virulent or temperate. Integration of temperate bacteriophages into the host genome during lysogenic life cycle renders the bacterial cell resistant to superinfection by the same type of temperate bacteriophage. This phenomenon, called immunity or homoimmunity, is the main reason why only virulent bacteriophages, displaying lytic life cycles leading to the lysis and death of the bacterial cell, are suitable for biocontrol applications. Furthermore, *Listeria* bacteriophages intended for biocontrol should be also safe for human health and for release into the environment.

Up to date, more than 500 *Listeria* bacteriophages have been isolated and identified. However, most of them have been reported as temperate bacteriophages and, therefore, discarded for biocontrol purposes (Hagens, S. and Loessner, M.J., 2014. Front Microbiol, 5: 159). Only few virulent bacteriophages, with potential for *Listeria* biocontrol, have been fully characterized at molecular and genomic level so far (Lasagabaster, A. et al., 2020. Food and Chemical Toxicology, 145, 111682).

The patent US7438901 B2 discloses the virulent (lytic) *L. monocytogenes* phage P100 from the *Myoviridae* family, which can be administered to food products, to the components that will be added to food products, and/or to the infrastructure of the food processing plants within which such food products are processed in order to reduce *L. monocytogenes* contamination. The patents US7507571 B2 and US8685697 B1 disclose isolated *L. monocytogenes* bacteriophages and the use thereof (alone or in combination) for the treatment of host infections or environmental contamination by *L. monocytogenes.* The use of bacteriophage cocktails/mixtures/formulations has also been proposed to limit the potential emergence of bacteriophage resistance and to obtain a broader specificity range against target bacteria. Nevertheless, the use of bacteriophage compositions disclosed in the above-mentioned patents (US7507571 B2 y US8685697 B1) still show a not broad enough activity against *L. monocytogenes* (<90% and <80% of tested strains, respectively), and/or no activity against all *L. monocytogenes* serotypes, and/or no activity against some other species within the genus *Listeria* that could be clinically relevant or act as food spoilage agents. Accordingly, it would be desirable to have materials, methods and processes that do not suffer from one or more of the above-mentioned drawbacks.

### DESCRIPTION OF THE INVENTION

The inventors of the present invention have isolated six bacteriophages which show a specific lytic capacity against a broad range of *Listeria* spp. further to be stable under a wide range of pH and temperature and do not include any known gene involved in lysogeny, toxin production, bacterial virulence or antibiotic resistance. The isolated bacteriophages are capable of lysing *L. monocytogenes* isolates belonging to lineage I, including the serotypes associated with most human clinical cases (1/2b and 4b), and lineage II, which harbour the serotypes most common in food products, natural and farm environments and associated with animal listeriosis cases and sporadic human clinical cases (1/2a). Advantageously, the bacteriophages isolated by the inventors are not only capable of lysing the major *L. monocytogenes* serotypes including, but not limiting to, serotypes 1/2a, 1/2b, 1/2c, 3a, 3b, 3c, 4a, 4b, 4c, 4d and 4e, but also other *Listeria* species, such as *Listeria ivanovii, Listeria seeligeri, Listeria welshimeri* or *Listeria innocua.* Furthermore, all bacteriophages are capable of infecting various prophage-free *Listeria* strains, including a non-pathogenic prophage-free *Listeria* strain, which could be safely used as propagation hosts for large industrial scale production.

Thus, thanks to the isolated bacteriophages of the present invention, it is ensured to reduce or eliminate *Listeria* spp. on food (including pet food), feed, beverage and different surfaces, being useful in biocontrol processes. They can be used for the treatment of listeriosis on humans and animals (therapy in aquaculture, livestock and other food-producing animals or pet animals); or used directly as additives/preservatives/processing aids in food (including pet food) and beverage industry, feed and animal feeding industry; or as sanitization and/or sterilization compositions to be applied on aquaculture facilities, slaughterhouses, animal transportation and/or food/feed processing industries; or for bacteria detection.

### Composition of the invention

Thus, in an aspect, the present invention relates to a composition comprising the bacteriophage AZT451 deposited in the Collection Nationale de Cultures de Microorganismes of Institut Pasteur (CNCM) with deposit number CNCM I-5799. Hereinafter, "composition of the invention".

As use herein, the terms "composition", "formulation", "cocktail" or "mixture" are considered equivalents and can be used equally throughout the present description. The composition may be liquid or dried form, such as a powder obtained after drying (e.g., spray-drying or lyophilization). Additional encapsulation and/or other stabilization processes, e.g. technology based on nanoparticles, may also be applied to the composition of the invention.

As used herein, the term "bacteriophage", also known as phage, refers to a virus that infects and replicates only in bacterial cells.

The bacteriophage AZT451 with deposit number CNCM I-5799 was isolated by standard techniques from a sample of cow faeces and deposited in the Collection Nationale de Cultures de Microorganismes of Institut Pasteur (CNCM) on December 16, 2021 under the Budapest Treaty. The depositor was AZTI (Address: Parque Tecnológico de Bizkaia, Astondo Bidea, Edificio 609, 48160 Derio (Bizkaia) Spain). The bacteriophage AZT451 belongs to *Caudovirales* Order, *Myoviridae* family, with an icosahedral capsid containing double stranded DNA and a contractile tail.

The composition of the invention may further comprise other bacteriophages, in particular, other bacteriophages isolated by the inventors which show similar features but with slightly different genomes and host range than the bacteriophage AZT451.

Thus, in a particular embodiment, the composition of the invention further comprises at least one, two, three, four and/or five bacteriophages selected from the group consisting of bacteriophage AZT356 with deposit number CNCM I-5797, bacteriophage AZT154 with deposit number CNCM I-5796, bacteriophage AZT450 with deposit number CNCM I-5798, bacteriophage AZT153 with deposit number CNCM I-5795, and bacteriophage AZT150 with deposit number CNCM I-5794.

The bacteriophage AZT356 with deposit number CNCM I-5797 was isolated by standard techniques from a sample of livestock environments and deposited in CNCM on December 16, 2021 under the Budapest Treaty. The depositor was AZTI (Address: Parque Tecnológico de Bizkaia, Astondo Bidea, Edificio 609, 48160 Derio (Bizkaia) Spain).

The bacteriophage AZT154 with deposit number CNCM I-5796 was isolated by standard techniques from a sample of cow faeces and deposited in CNCM on December 16, 2021 under the Budapest Treaty. The depositor was AZTI (Address: Parque Tecnológico de Bizkaia, Astondo Bidea, Edificio 609, 48160 Derio (Bizkaia) Spain).

The bacteriophage AZT450 with deposit number CNCM I-5798 was isolated by standard techniques from a sample of cooked ham and deposited in CNCM on December 16, 2021 under the Budapest Treaty. The depositor was AZTI (Address: Parque Tecnológico de Bizkaia, Astondo Bidea, Edificio 609, 48160 Derio (Bizkaia) Spain).

The bacteriophage AZT153 with deposit number CNCM I-5795 was isolated by standard techniques from a sample of sheep faeces and deposited in CNCM on December 16, 2021 under the Budapest Treaty. The depositor was AZTI (Address: Parque Tecnológico de Bizkaia, Astondo Bidea, Edificio 609, 48160 Derio (Bizkaia) Spain).

The bacteriophage AZT150 with deposit number CNCM I-5794 was isolated by standard techniques from a sample of sheep faeces and deposited in CNCM on December 16, 2021 under the Budapest Treaty. The depositor was AZTI (Address: Parque Tecnológico de Bizkaia, Astondo Bidea, Edificio 609, 48160 Derio (Bizkaia) Spain).

All these bacteriophages belong to *Caudovirales* Order, *Myoviridae* family, with an icosahedral capsid containing double stranded DNA and a contractile tail.

The invention further contemplates "variants" of these bacteriophages (bacteriophage CNCM I-5794, bacteriophage CNCM I-5795, bacteriophage CNCM I-5796, and bacteriophage CNCM I-5797, bacteriophage CNCM I-5798, and bacteriophage CNCM I-5799), which are bacteriophages having minor variation(s) in the genomic sequence and polypeptides encoded thereby while retaining the same general genotypic and phenotypic characteristics as the wild-type bacteriophage. An assay for checking if a given bacteriophage is a variant of the above bacteriophages is disclosed in the Examples of the present description. Variants of the bacteriophages encompass polymorphic variants. The invention also contemplates "derivative" bacteriophages, which are bacteriophages having modified genotypic or phenotypic characteristics relative to the above-mentioned deposited bacteriophages. Derivative bacteriophages of the invention particularly encompass designed bacteriophages harboring genes encoding novel phenotypic traits. Such recombinant bacteriophages are engineered to contain novel genes having traits not found in wild-type bacteriophage.

In another particular embodiment, the composition of the invention comprises at least two bacteriophages, or variants thereof, being said bacteriophages
▪ Bacteriophage CNCM I-5799 and bacteriophage CNCM I-5797, or
▪ Bacteriophage CNCM I-5799 and bacteriophage CNCM I-5796, or
▪ Bacteriophage CNCM I-5799 and bacteriophage CNCM I-5798, or
▪ Bacteriophage CNCM I-5799 and bacteriophage CNCM I-5795, or
▪ Bacteriophage CNCM I-5799 and bacteriophage CNCM I-5794.

In another particular embodiment, the composition of the invention comprises at least three bacteriophages, or variants thereof, being said bacteriophages:
▪ Bacteriophage CNCM I-5799, Bacteriophage CNCM I-5797, and Bacteriophage CNCM I-5796, or
▪ Bacteriophage CNCM I-5799, Bacteriophage CNCM I-5797, and Bacteriophage CNCM I-5798, or
▪ Bacteriophage CNCM I-5799, Bacteriophage CNCM I-5797, and Bacteriophage CNCM I-5795, or
▪ Bacteriophage CNCM I-5799, Bacteriophage CNCM I-5797, and Bacteriophage CNCM I-5794, or
▪ Bacteriophage CNCM I-5799, Bacteriophage CNCM I-5794, and Bacteriophage CNCM I-5798, or
▪ Bacteriophage CNCM I-5799, Bacteriophage CNCM I-5794, and Bacteriophage CNCM I-5795, or
▪ Bacteriophage CNCM I-5799, Bacteriophage CNCM I-5794, and Bacteriophage CNCM I-5794, or
▪ Bacteriophage CNCM I-5799, Bacteriophage CNCM I-5798, and Bacteriophage CNCM I-5794, or
▪ Bacteriophage CNCM I-5799, Bacteriophage CNCM I-5798, and Bacteriophage CNCM I-5794, or
▪ Bacteriophage CNCM I-5799, Bacteriophage CNCM I-5795, and Bacteriophage CNCM I-5794.

In another particular embodiment, the composition of the invention comprises at least four bacteriophages, or variants thereof, being said bacteriophages:
▪ Bacteriophage CNCM I-5799, Bacteriophage CNCM I-5797, Bacteriophage CNCM I-5795, and Bacteriophage CNCM I-5798, or
▪ Bacteriophage CNCM I-5799, Bacteriophage CNCM I-5797, Bacteriophage CNCM I-5796, and Bacteriophage CNCM I-5795, or
▪ Bacteriophage CNCM I-5799, Bacteriophage CNCM I-5797, Bacteriophage CNCM I-5796, and Bacteriophage CNCM I-5794, or
▪ Bacteriophage CNCM I-5799, Bacteriophage CNCM I-5797, Bacteriophage CNCM I-5798, and Bacteriophage CNCM I-5795, or
▪ Bacteriophage CNCM I-5799, Bacteriophage CNCM I-5797, Bacteriophage CNCM I-5798, and Bacteriophage CNCM I-5794, or
▪ Bacteriophage CNCM I-5799, Bacteriophage CNCM I-5797, Bacteriophage CNCM I-5795, and Bacteriophage CNCM I-5794, or
▪ Bacteriophage CNCM I-5799, Bacteriophage CNCM I-5796, Bacteriophage CNCM I-5798, and Bacteriophage CNCM I-5795, or
▪ Bacteriophage CNCM I-5799, Bacteriophage CNCM I-5796, Bacteriophage CNCM I-5798, and Bacteriophage CNCM I-5794, or
▪ Bacteriophage CNCM I-5799, Bacteriophage CNCM I-5796, Bacteriophage CNCM I-5795, and Bacteriophage CNCM I-5794, or
▪ Bacteriophage CNCM I-5799, Bacteriophage CNCM I-5798, Bacteriophage CNCM I-5795, and Bacteriophage CNCM I-5794.

In another particular embodiment, the composition of the invention comprises at least five bacteriophages, or variants thereof, being said bacteriophages:
▪ Bacteriophage CNCM I-5799, Bacteriophage CNCM I-5797, Bacteriophage CNCM I-5796, Bacteriophage CNCM I-5798, and Bacteriophage CNCM I-5795, or
▪ Bacteriophage CNCM I-5799, Bacteriophage CNCM I-5797, Bacteriophage CNCM I-5796, Bacteriophage CNCM I-5798, and Bacteriophage CNCM I-5794, or
▪ Bacteriophage CNCM I-5799, Bacteriophage CNCM I-5797, Bacteriophage CNCM I-5796, Bacteriophage CNCM I-5795, and Bacteriophage CNCM I-5794, or
▪ Bacteriophage CNCM I-5799, Bacteriophage CNCM I-5797, Bacteriophage CNCM I-5798, Bacteriophage CNCM I-5795, and Bacteriophage CNCM I-5794, or
▪ Bacteriophage CNCM I-5799, Bacteriophage CNCM I-5796, Bacteriophage CNCM I-5798, Bacteriophage CNCM I-5795, and Bacteriophage CNCM I-5794.

In another particular embodiment, the composition of the invention comprises at least six bacteriophages, or variants thereof, being said bacteriophages: Bacteriophage CNCM I-5799, bacteriophage CNCM I-5797, bacteriophage CNCM I-5796, bacteriophage CNCM I-5798, bacteriophage CNCM I-5795, and bacteriophage CNCM I-5794.

As the skilled person in the art understands, the composition of the invention may also comprise one or more further compounds. Thus, in particular embodiment of the composition of the invention, alone or in combination with all or each one of the previous particular embodiments, the composition further comprises a carrier and/or an organic acid or salt thereof.

As use herein, the term "carrier" refers to a compound (such as a diluent, an adjuvant, an excipient, or a vehicle) which facilitates the incorporation of other compounds to allow a better dosing and administration or to give consistency and form to the composition. Therefore, the carrier is a substance which is used to dilute any of the components of the composition of the present invention to a determined volume or weight, or even without diluting said components, capable of allowing better dosing and administration or giving consistency. When the form of presentation is liquid, the carrier is the diluent. As the skilled person in the art understands, the carrier must not compromise the viability of the bacteriophage of the composition and be non-toxic to any human or non-human animal. In a particular embodiment of the composition of the invention, alone or in combination with all or each one of the previous particular embodiments, the carrier is (i) a pharmaceutically acceptable carrier (both for veterinary and human use), or (ii) a food acceptable carrier, or a biotechnologically acceptable carrier. Examples of these carriers are known in the state of the art.

As use herein, the term "organic acid" refers to any compound comprising one or more carboxylic acid groups. Examples of organic acids include, without being limited to, lactic acid, acetic acid, propionic acid and mixtures thereof. Examples of salts of the organic acid include, without being limited to, sodium salt, potassium salt, ammonium salt and mixtures thereof, such as K-(L)lactate, Na-(L)lactate, K-acetate, Na-acetate, K-diacetate, Na-diacetate, K-propionate, Na-propionate and mixtures thereof. The organic acid may be acetic acid in a buffered aqueous solution comprising 2% to 30% acetate, such as comprising 5% to 20% acetate, such as comprising 10% to 20% acetate, such as comprising 15% to 20% acetate, such as comprising 15, 16, 17, 18, 19 or 20% acetate, such as comprising 17% acetate. The pH of the acetic acid in a buffered aqueous solution may be 2 to 7, such as pH 5 to 6.5, such as pH 5.7 to 6.3. Buffering may be performed using sodium acetate, acetic acid, sodium hydroxide, sodium carbonate and/or sodium bicarbonate.

In another particular embodiment, alone or in combination with all or each one of the previous particular embodiments, the composition further comprises one or various bacteriophage-derived proteins, surface disinfectants, antibiotics, surfactants, enzymes or multi-enzyme formulations, additives, preservatives, processing aids, protective cultures, further *Listeria* specific bacteriophages and/or further bacteriophages with specific activity against bacterial contaminants other than *Listeria* spp.

As used herein, the term "bacteriophage-derived proteins" refers to both "bacteriophage-associated polysaccharide degrading enzymes" and "bacteriophage-derived proteins involved in progeny release":
Polysaccharide-degrading enzymes, also referred to as "polysaccharide depolymerases", are bacteriophage-associated proteins employed to enzymatically degrade the capsular (alginate, hyaluronan, polysialic acid, amylovoran) or structural (lipopolysaccharide, peptidoglycan) polysaccharides of the bacterial host at the first step of the phage infection process. Polysaccharide depolymerases are widely known in the state of the art and any of them can be used in the present invention. Examples of such enzymes include, without limiting to, enzymes of the following five major groups: peptidoglycan hydrolases (such as lysozymes, lytic transglycosylases, glucosaminidases, endopeptidases, etc), endosialidases (also termed endo-N-acetylneuramini- dases), endorhamnosidases, alginate lyases and hyaluronate lyases.

Bacteriophage-derived proteins involved in progeny release refer to hydrolytic enzymes produced by bacteriophages in order to cleave the host's cell wall during the final stage of the lytic cycle. Examples of such enzymes include, without limiting to, enzymes of the following three major groups: "holins", "endolysins" (also known as "lysins" or "murein hydrolases"), and "spanins", responsible for destruction of the inner membrane, destruction of murein, and crossing the outer membrane, respectively. Holins, endolysins and spanins are widely known in the state of the art and any of them can be used in the present invention. Examples of holins include, without limiting to, HolSMP protein, encoded by *Streptococcus* suis phage SMP, P68 hol15 derived from *Staphylococcus aureus* phage P68, S protein encoded by *Enterobacteria* phage λ, pinholin S encoded by coliphage 21 or Hol3626 protein from *Clostridium perfringens* bacteriophage Φ3626. Examples of endolysin include, without limiting to, endolysin Cpl-1 from Cp-2 phage, endolysin PAL from DP-1 phage, endolysin PlyC from phage C1, endolysin PlyGBS from phage NTCN 11364, endolysin PlyG from phage γ, endolysin PlyPH from prophage of the *Bacillus anthracis* Ames strain, endolysin MV-L from phage MR11, endolysin CHAPκ from phage K and endolysin LysGH15 from phage GH15. An example of endolysins are listeriolysins which are enzymes that have been shown to selectively control *Listeria* in food and the environment. Examples of spanins include, without limiting to, Rz i-spanin or Rz1 o-spanin from λ phage, gp11 u-spanin encoded by *E. coli* phage.

Examples of surface disinfectants include, but not limited to, (i) preservatives of various kinds, such as benzoic acid or BHT; (ii) iodophors that provide iodine; and (iii) disinfectants with which the phages are compatible, such as hypochlorite, halogenated agents, alcohols, anionic acids, amphoteric agents, phenolics and quaternary ammonium compounds.

Examples of antibiotics include, without limiting to, amikacin, ampicillin, amoxicillin, azithromycin, carbenicillin, ceftazidime, chloramphenicol, ciprofloxacin, cefoperazone, colistin, danofloxacin, erythromycin, fosfomycin, gentamycin, kanamycin, linezolid, meropenem, neomycin, nisin, piperacillin, tetracycline or vancomycin.

Examples of enzymes include, without limiting to, antimicrobial enzymes and multi-enzyme formulations aimed to degrade bacteria biofilms, such as polysaccharide depolymerases, proteases, carbohydrases, oxidoreductases or anti-quorum sensing enzymes.

The composition of the invention may comprise surfactants when used to treat food processing equipment. The surfactant helps to wet the surface so that the phage is properly distributed over the various surfaces, and to solubilize and remove dirt so that the *Listeria* spp. are accessible to the phage. Suitable surfactants include, but not limited to, Tween 80, Tween 20, Tween 81 and Dobanols.

The composition of the invention may comprise food, feed or pharmaceutical additives, preservatives, and/or processing aids.

Examples of additives are widely known in the state of the art and include, without limiting to:
- colouring additives (such as carotenes, lycopene, lutein, curcumin, riboflavin, tartrazine, carmines, amaranth, erythrosine, chlorophylls, caramel, calcium carbonate, aluminium, silver, gold, etc.),
- sweeteners (such as sorbitol, mannitol, acesulfame K, aspartame, isomalt, saccharin, sucralose, steviol, neotame, maltitol, lactitol, xylitol, etc.),
- other approved additives (such as acetic acid and acetate salts, lactic acid and lactate salts, carbon dioxide, malic acid, fumaric acid, citric acid and citrate salts, tartaric acid and tartrate salts, phosphoric acid and phosphate salts, diphosphates, triphosphates, malate salts, metatartaric acid, adipic acid and adipate salts, succinic acid, glycerol, carbonate salts, hydrochloric acid and chloride salts, sulphuric acid and sulphate salts, sodium hydroxide and hydroxide salts, calcium or magnesium oxide, ferrocyanide salts, silicon dioxide, silicates, talc, fatty acids, gluconic acid, gluconates, glutamic acid, glutamates, waxes, etc.),
- nutritional additives (such as vitamins and minerals), zootechnical additives (such as amylases, cellulases, xylanases, glucanases, protective cultures, etc.),
- emulsifiers, stabilisers, thickeners and gellind agents (such as starchs, lecithins, alginic acid and alginates, agar, carrageenan, carob gum, guar gum, tragacanth, gum arabic, xantham gum, karaya gum, konjac, cassia gum, polysorbates, pectins, celluloses, salts of fatty acids, sucroglycerides, lactylate salts, invertase, etc.), and
- coccidiostats and histomonostats as approved for animal feed (such as salinomycin, monensin, decoquinate, robenidine hydrochloride, lasalocid A, halofuginone, narasin, maduramicin, etc.), etc.

Examples of preservatives are widely known in the state of the art and include, without limiting to:
- preservatives (such as sorbic acid, potassium sorbate, benzoic acid and benzoate salts, sulphur dioxide and sulphite salts, nisin, natamycin, nitrite and nitrate salts, propionic acid and propionate salts, boric acid, lysozyme, butylparaben, etc.), and
- antioxidants (such as ascorbic acid and ascorbates, tocopherols, propyl gallate, erythorbic acid, TBHQ, BHA, BHT, etc.).

Examples of processing aids are widely known in the state of the art and include, without limiting to:
- anti-foaming agents (such as silicone, silicone dioxide, polyethylene glycol, dimethylpolysiloxane, alginates, polysorbates, ammonium phosphatides, fatty acids, etc.),
- antimicrobial and decontaminating agents (such as ozone, chlorine organic acid, ammonium hydroxide, calcium chloride, hydrogen peroxide, peroxyacid, ascorbic acid, acetic acid, peracetic acid, etc.),
- anti-adherent agents (such as silica, talc, magnesium stearate, etc.),
- clarifying and bleaching agents (such as citric acid and its sodium salts, tartaric acid and its sodium salts, phosphoric acid and its sodium salts, bentonite, activated carbon/charcoal, silicon dioxide, albumin, gelatin, casein, keratin, etc.),
- filtering agents (such as diatomaceous earths, cellulose, polyamides, etc), and
- packing gases (such as nitrogen, helium, carbon dioxide, argon, etc).

The composition of the invention may also comprise protective cultures relating to bacteria and yeasts that are able to inhibit the growth of target non-desired bacteria through competition for nutritive ingredients and/or production of one or more antimicrobial metabolites (e.g., organic acids, hydrogen peroxide, antimicrobial enzymes, bacteriocins, reuterin etc.). Examples of protective cultures include, without limiting to, specific strains of *Lactobacillus brevis, Lactobacillus plantarum, Lactobacillus acidophilus, Lactobacillus rhamnosus, Lactobacillus buchneri, Lactobacillus casei, Lactobacillus reuteri, Lactobacillus kefiri, Lactobacillus paracasei, Lactobacillus farciminis, Enterococcus faecium, Bacillus subtilis, Bacillus licheniformis, Lactococcus lactis, Pediococcus acidilactici, Pediococcus pentosaceus, Propionibacterium acidipropionici, Saccharomyces cerevisiae,* etc.

The composition may also comprise further *Listeria* bacteriophages, preferably L. *monocytogenes* specific bacteriophages, different from the phages of the invention. Examples of these phages are widely known in the state of the art and include, without limiting to, P61, P100, P200, P825, List-1, List-36, List-38, LMSP-25, LMTA-57, LMTA-94, LMTA-148, LMPC01, LMPC02, and LMPC03, LP-125 or LP-064 bacteriophages.

The composition may also comprise further bacteriophages specific for spoilage and/or pathogenic bacteria other than *Listeria* spp. A further bacteriophage can be any phage known in literature with specific activity against any target bacteria not belonging to *Listeria* genus. Examples include, without limiting to, *Aeromonas, Campylobacter, Clostridium, Escherichia, Klebsiella, Proteus, Pseudomonas, Salmonella, Shigella, Staphylococci, Streptococci,* or *Vibrio* specific bacteriophages.

Alternatively, in case that all the components listed above were not present in the composition of the invention, they can be administered simultaneously, previously or sequentially to said composition comprising the bacteriophage/s of the invention.

As the skilled person in the art understands, the composition of the invention can be also administered simultaneously, previously or sequentially to one or more traditional and/or innovative "food processing technologies". "Food Processing Technology" refers to any physical, chemical, or microbiological method or technique used to transmute/transform raw ingredients into food products, including those preservation/decontamination technologies aiming to improve the microbiological quality and safety of the final food products. Examples of food processing technologies include, without limiting to, cooking, thermal pasteurization and sterilization, high pressure processing, electrical heating, cold plasma, infrared processing, microwave technology, ultraviolet light, pulsed light, ultrafiltration, pulsed electric fields, ultrasound processing, drying technologies and freezing technologies.

The composition of the invention may comprise any amount of bacteriophage. Nevertheless, in a particular embodiment of the composition of the invention, alone or in combination with all or each one of the previous particular embodiments, the bacteriophage as disclosed herein is present in an aqueous liquid and comprises from 1×10⁶ plaque forming units (PFU)/mL to 1×10¹² PFU/mL, in particular, from 1×10⁷ PFU/mL to 1×10¹¹ PFU/mL, in particular, from 1×10⁸ PFU/mL to 1×10¹⁰ PFU/ml, in particular 1×10⁹ PFU/mL. In a more particular embodiment, alone or in combination with all or each one of the previous particular embodiments, the bacteriophage as disclosed herein is present in an aqueous liquid and comprises 1×10⁶ PFU/mL, 1×10⁷ PFU/mL, 1×10⁸ PFU/mL, 1×10⁹ PFU/mL, 1×10¹⁰ PFU/mL, 1×10¹¹ PFU/mL, or 1×10¹² PFU/mL. The person skilled in the art knows how to calculate and assay PFUs.

### Uses of the composition of the invention

As explained in the beginning of the present description, the bacteriophages isolated by the inventors show an improved lytic capacity which make them suitable to eliminate *Listeria* spp. on food, feed, beverage and different surfaces, being useful in biocontrol processes.

Thus, in another aspect, the present invention relates to the use of the composition of the invention for controlling, reducing and/or eliminating *Listeria* spp. contamination, or for preventing the growth or colonization of *Listeria* spp., in food- or feed-products, in pharmaceutical, veterinary or biotechnological products, in water systems, in fluids, in soils, or in any non-food surfaces. Hereinafter "first use of the invention".

As used herein, the term "controlling", "reducing" or "eliminating" refers to any measurable decrease in the amount of *Listeria* spp. with respect to the initial amount of *Listeria* spp. Particularly, the term "controlling" or "reducing" means a decrease by 10%, 20%, 30%, 40%, 50%, 60%, 70%, 80%, 90%, 95%, 98% and/or 99% of the original bacterial count. In the case of food, the decrease of the original bacteria count is up to the minimum amount of *Listeria* spp. which can be present in a food to be considered suitable for human intake. This minimum amount of *Listeria* spp. is laid down by the International/National Regulations widely known in the state of the art, such as the Commission Regulation (EC) No 2073/2005 of 15 November 2005 in force at the moment of filling the present patent application. Preferably, no *Listeria* spp. is detectable. The expression "amount of *Listeria* spp." means viable *Listeria* spp. bacteria. *Listeria* spp. may be detected using standard techniques known by the person skilled in the art such as immunohistochemical techniques using *Listeria* spp. specific antibodies such as immunomagnetic separation, agglutination and enzymelinked immune assays, immunochromatography or fluorescence detection; Viable *Listeria* spp. may be detected using standard techniques known by the person skilled in the art such as microbiological bacterial culture techniques and/or real-time quantitative transcription polymerase chain reaction to assay for bacterial nucleic acid.

As used herein, the term "contamination" encompasses bacterial growth, colonization, or infection, and any combination thereof, of bacterial pathogens, particularly, in the present invention the bacterial pathogen is *Listeria* spp.

As use herein, the term "preventing the growth of *Listeria* spp." or "preventing the colonization of *Listeria* spp." refers to the intention of providing a food- or feed-product, a pharmaceutical, veterinary or biotechnological product, a water system, a fluid, or a non-food surface to be devoid of, or contains minimal numbers of, viable *Listeria* spp. that could cause illness in humans or animals or spoilage of the product prior to ingestion / administration.

In line with the foregoing terms "controlling", "reducing" or "eliminating", the prevention of growth of *Listeria* spp. on the above-mentioned places is also intended to include, but is not limited to, the following mechanisms: (1) removal of *Listeria* spp. from the food products or the non-food surfaces; (2) inhibition of attachment of *Listeria* spp. to the food products or to the non-food surfaces; (3) killing or inactivation of *Listeria* spp. on/in the food products or the non-food surfaces; and (4) killing or inactivation of microorganisms which are not in/on the food product or the non-food surfaces, but which are present in liquids associated with food products during processing; such as in chill tanks, or which are present on surfaces associated with food preparation, liquids remaining on such surfaces, such as countertops, cutting boards and sinks, and equipment used in food preparation and sanitization of the food.

The food product may be any food product that is known in the art and is susceptible to *Listeria* spp. contamination and/or spoilage. The food product may be a processed, non-processed, cured or uncured food product selected from the group consisting of meat, fish, shellfish, pastry, dairy product, vegetables, fruit and mixtures thereof. In an embodiment, the food product is selected from the group consisting of beef, pork, lamb, fruit, vegetables, including but not limited to lettuce, leafy greens, baby leafy greens, sprouts. The processing or curing of the food product may be a process selected from the group consisting of cooking, salting, baking, steaming, smoking, grilling, roasting, drying and brining (e.g., injecting with brine).

Thus, in a particular embodiment of the first use of the invention, the food product is a "ready-to-eat" product, a dairy product, an unpasteurized food product, a vegetable, a meat, or a fish. In another particular embodiment, the feed-product is fodder.

As used herein, the term "ready-to-eat" product is intended to include any food product intended by its producer or manufacturer to direct consumption without the need of cooking or another kind of transformation efficient in eliminating or reducing the pathogenic microorganisms present in the product to an acceptable level.

As used herein, the term "dairy product" is intended to include any food product made using milk or milk products, including but not limited to milk, yogurt, ice cream, cheese, butter, and cream.

As used herein, the term "unpasteurized food product" is intended to include any food product which is prepared using unpasteurized primary ingredients and which does not undergo a final (listericidal) heat treatment.

As used herein, the term "meat product" is intended to include any food product which contains animal tissue, including but not limited to, poultry, such as chicken and turkey, beef, pig/pork, horse, donkey, rabbit, goat, sheep, sausage, such as frankfurter, bologna, meatloaf, roast beef, ham, sliced meat and mixtures thereof, as known in the art.

As used herein, the term "fish product" is intended to include any food product which contains tissue from an aquatic animal including, but not limited to, salmon, cod, trout, lobster, crawfish, crab, fresh water and saltwater fish and other seafoods such as clamps or shrimps.

As used herein, the term "vegetable" is intended to include any food product which contains tissues from plants that are consumed by humans or other animals as food. Examples of vegetables include, but not limited to, fruits, cabbage, Brussels sprouts, cauliflower, broccoli, kale, kohlrabi, red cabbage, Savoy cabbage, Chinese broccoli, collard greens, turnip, Chinese cabbage, napa cabbage, bok choy, radish, daikon, seedpod varieties, carrot, parsnip, beetroot, sea beet, Swiss chard, sugar beet, lettuce, celtuce, green bean, French bean, runner bean, haricot bean, Lima bean, broad bean, pea, snap pea, snow pea, split pea, eggplant (aubergine), tomato, sweet potato, cucumber, pumpkin, squash, marrow, zucchini (courgette), gourd onion, spring onion, scallion, shallot, garlic, leek, elephant garlic, pepper, bell pepper, sweet pepper, spinach, yam and cassava. In the present invention, the term "vegetable" is intended to include "salad", i.e., any food product which contains mixtures of vegetables or fruits, and particularly such mixtures as are presented for consumers to choose from in a display commonly referred to as a "salad bar".

As used herein, the term "fodder" refers to any agricultural foodstuff used specifically to feed domesticated livestock, such as cattle, rabbits, sheep, horses, chickens and pigs. "Fodder" refers particularly to food given to the animals (including plants cut and carried to them), rather than that which they forage for themselves (called forage). Examples of fodder includes, without limiting to, hay, straw, silage, compressed and pelleted feeds, oils and mixed rations, and sprouted grains and legumes (such as bean sprouts, fresh malt, or spent malt).

The composition of the invention may be administered to the food product in any way known to the person skilled in the art, such as by mixing with the food product or dipping the food product in the composition of the invention, by spraying or misting composition of the invention on/over the food product, or by including the composition of the invention in/on the packaging materials. The person skilled in the art knows to select a proper method.

As indicated above, the present invention also encompasses the use of the composition of the invention for controlling, reducing and/or eliminating *Listeria* spp. contamination, or for preventing the growth or colonization of *Listeria* spp. in pharmaceutical, veterinary or biotechnological products. As used herein: the term "pharmaceutical product" relates to any product comprising drugs useful in the treatment of a disease in a human subject; the term "veterinary product" relates to any product comprising drugs useful in the treatment of a disease in a non-human subject, and the term "biotechnical product" relates to any product obtained by the use of microorganisms, or parts thereof, through industrial processes.

Likewise, the present invention also encompasses the use of the composition of the invention for controlling, reducing and/or eliminating *Listeria* spp. contamination, or for preventing the growth or colonization of *Listeria* spp. in a water system. Thus, in another particular embodiment of the first use of the invention, alone or in combination with all or each one of the previous particular embodiments, the water system include, without limiting to, livestock drinking water, poultry chiller water, brines, hydro-coolers, brine coolers or chillers, cleaning water, baths and flumes for fruits and vegetables and sewage.

For use in a water system, the composition of the invention may be available in several forms, including, but not limited to, a liquid, a tablet or a powder. Any of these forms may be added directly to the water system. Alternatively, it may be preferred to premix and dilute the composition before adding it to the water system, in which case other components may easily be added during dilution. The other components may include a buffering agent to control the pH level and/or other adjuvants as mentioned above.

The composition of the invention may be added to the water system either manually or automatically. A control system may be used to monitor a concentration of bacteriophage in the water system to ensure that an effective concentration of the bacteriophage is maintained. The control system may be configured such that a replenishment bacteriophage is added to the water system periodically as a function of time or based on sensed parameters within the water system. The control system may also monitor a pH of the water system to ensure that the water system is maintained at a pH range that ensures viability of the bacteriophage.

Treating the water with the composition of the invention creates another opportunity to reduce bacterial contamination. Food products benefit from using the water treatment in combination with applying the bacteriophage directly to the food product. For example, in poultry chiller water, there is the potential for a first piece of poultry to contaminate the water; then, when a second piece of poultry is placed in the water, the second piece of poultry may become contaminated. By eliminating the bacteria in the water from the first piece of poultry, the bacteriophage has the potential to stop the spread of bacteria during the processing steps.

In another example, cleaning of fruits and/or vegetables may involve placing the fruits or vegetables in a flume of water. By treating the water in the flume with the composition of the invention, it is possible to control, reduce and/or eliminate *Listeria* spp. contamination in the flume. Moreover, the treatment of the flume may be used in combination with other bacteriophage treatments for fruits and vegetables. As described above, these may include any of spraying the crops with the composition of the invention prior to harvest, spraying the fruits and vegetables before shipping to a retailer, and/or spraying the fruits and vegetables during or after stocking at the retail store.

Although specific applications for a water system are described above, it is recognized that the composition of the invention may be applied to essentially any water system having a potential for *Listeria* spp. contamination.

The composition of the invention may also be used for non-food surfaces. Equipment used for food processing, as well as the surrounding areas, may commonly be a source of *Listeria* spp. contamination. Thus, in another particular embodiment of the first use of the invention, alone or in combination with all or each one of the previous particular embodiments, the non-food surface is
- the surface of food, feed, pharmaceutical, veterinary or biotechnological processing equipment,
- the surface of food, feed, pharmaceutical, veterinary or biotechnological storage containers,
- the surface of pipes,
- soil,
- the surface of animal bedding,
- the surface of troughs, or
- the surface of an animal transportation container.

The composition of the invention may be applied to any non-food surface provided that the bacteriophage is delivered in a manner to ensure proper contact between the bacteriophage and the surface. The bacteriophage may be applied, for example, to any of the equipment in a deli, such as meat slicing equipment, including a blade. The composition of the invention also may be applied to floors, walls, sinks and drains of the deli.

The composition of the invention may be applied using any of the delivery methods described above, so long as the bacteriophage of the composition of the invention adequately covers and adheres to the intended surface. In some cases, the composition may be applied as a thickened layer. If the composition is to be applied to a generally vertical surface, it is preferred that a thickened treatment is used. Thickeners or gel forming agents may be used to create the thickened composition. An appropriate concentration of the thickening agent is between approximately 100 ppm and 10 weight percent. In a preferred embodiment, the concentration of the thickening agent is between approximately 100 ppm and 2.0 weight percent.

Alternatively, the composition of the invention may be applied as a foam to the surface. In that case, a foaming surfactant may be used at a concentration ranging between approximately 25 ppm and approximately 2.0 weight percent. The composition also may be sprayed onto the surface.

Examples of non-food surfaces include, but are not limited to, floor drains, sink drains, drip pans, cooler floors and walls, refrigerated cases, deli counters, deli walls, deli floors, forklifts, carts, tanks and tubs. Other examples of food processing equipment include, but not limiting to, a tube through which milk is being pumped, a high-salt content tank for processing cheese, a container from which cultures are applied to a surface of a cheese, a storage shelf for a food product, a shelf on which a food product is dried and cured, and a floor drain. Examples of food storage containers include, without limiting to, any packaging material, tanks, containers, hoppers, fermenters, and bioreactors.

The composition of the invention may also be added to any type of processing equipment or handling equipment used in the food (both human and veterinary food), feed, or beverage industry. The machinery in the food and beverage industry may commonly use food-grade greases, which may be treated with the composition of the invention. In other examples, red meat chill sprays and brine chill systems may also be treated with the composition of the invention.

By applying the composition of the invention to the equipment and other surfaces that the food may directly or indirectly come into contact with the bacteriophage, has a potential to greatly reduce the spread of bacteria in a food processing or handling facility. Moreover, the composition of the invention could be used at a consumer level as well. For example, a spray bottle may be used to spray the bacteriophage on kitchen counters and in sinks, stoves, ovens, refrigerators, etc.

Likewise, the composition of the invention may be used directly as an additive, preservative or processing aid in food and beverage industry, feed and animal feeding industry, every sort of hand, dental and facial cleansing materials and even in pharmaceutical industry.

Thus, in another aspect, the present invention relates to the use of the composition of the invention as an additive or preservative or processing aid in a food- or feed-product, in a beverage, or in pharmaceutical, veterinary, or biotechnological products. Hereinafter, second use of the invention.

In a particular embodiment of the second use of the invention, the food product is a "ready-to-eat" product, a dairy product, an unpasteurized food product, a vegetable, a meat, or a fish.

The terms "food product", "ready to eat product", "dairy product", "an unpasteurized food product", "a vegetable", "a meat", or "a fish product" has been defined above for the first use of the invention, and they are applicable to the second use of the invention. Likewise, the particular embodiments of the first use of the invention are also applicable to the second use of the invention. Thus, in another particular embodiment of the second use of the invention, the feed-product is fodder.

In another aspect, the present invention relates to the composition of the invention for use as a medicament. Hereinafter "first medical use of the invention".

The term "medicament" as used herein means a pharmaceutical composition suitable for administration of the active compound, in the present invention the active compound is composition of the invention, to both human and non-human subjects.

In another aspect, the present invention relates to the composition of the invention for use in the treatment of a subject infected with bacteria from *Listeria* ssp, preferably, *L. monocytogenes, L. innocua, L. ivanovii, L. seeligeri,* or *L. welshimeri.* Hereinafter "second medical use of the invention".

The term "treatment" as used herein refers to the treatment of a disease or medical condition in a subject, preferably human subject, which includes:
(a) preventing the disease or medical condition from occurring, i.e., prophylactic treatment of a subject;
(b) ameliorating the disease or medical condition, i.e., causing regression of the disease or medical condition in a subject;
(c) suppressing the disease or medical condition, i.e., slowing the development of the disease or medical condition in a subject; or
(d) alleviating the symptoms of the disease or medical condition in a subject.

In the context of the present invention, the disease or medical condition is an infection of *Listeria* ssp., preferably, an infection of *L. monocytogenes,* more preferably, an infection of *L. monocytogenes* serotypes 1/2a, 1/2b, 1/2c, 3a, 3b, 3c, 4a, 4b, 4c, 4d and 4e.

In the present invention, the subject to be treated can be any animal. As use herein, the term "subject" is equivalent to the term "individual"; whereby both terms can be used interchangeably herein. The term "subject" means any individual, any animal belonging to any species (including but not limited to humans). Examples of subjects include, but are not limited to, animals of commercial interest such as fish (trout, sardines, carp, salmon, etc.), poultry (chickens, ostriches, chickens, geese, quail, etc.), rabbits, hares, domestic animals (dogs, cats, etc.), ovine and caprine livestock (sheep, goats, etc.), porcine livestock (boars, pigs, etc.), equine livestock (donkeys, horses, ponies, etc.), or bovine livestock (bulls, oxen, etc.); animals of cynegetic interest, i.e., game such as deer, reindeer, etc .; animals of ecological interest, i.e., animals that are endangered or whose populations are scarce in nature, for example, white tigers, rhinos, pandas, cougars, lynx, etc., and humans. In a particular embodiment of the second medical use of the invention, alone or in combination with all or each one of the previous particular embodiments, the animal to be treated is a mammal, more preferably, a primate, still more preferably, a human of any sex, race or age.

As the skilled person in the art understands, in both the first and the second medical uses of the invention, the composition of the invention is formulated to be a pharmaceutical composition, hereinafter "pharmaceutical composition of the invention".

According to the conventional techniques known to those skilled in the art, the pharmaceutical composition of the invention may be formulated with a pharmaceutically acceptable excipient and/or carrier.

The term "carrier" has been defined previously herein. The term "excipient" refers to a substance which helps to absorb any of the components of the composition of the invention, stabilizes said components or helps in the preparation of the composition in the sense of giving it consistency or, if necessary, providing flavors which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material included in the galenic forms which is added to the active ingredients or to its associations to enable its preparation and stability, modify its organoleptic properties or determine the physical/chemical properties of the composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerin, amongst others.

The terms "pharmaceutically acceptable excipient" or a "pharmaceutically acceptable carrier" mean that the carrier or the excipient should allow for the activity of the compounds of the pharmaceutical composition (herein bacteriophages), that is to say, for it to be compatible with said components. Likewise, the composition is said to be "pharmacologically acceptable" if its administration can be tolerated and non-toxic by a recipient subject.

The pharmaceutical composition of the invention for use in the treatment of an infection of *Listeria* spp., may be administered by any appropriate administration route, to this end, said composition will be formulated in the suitable pharmaceutical form for the selected administration route. Suitable routes of administration may, for example, include depot, transdermal, oral, rectal, transmucosal, or intestinal administration; parenteral delivery, including intramuscular, subcutaneous, intravenous, intramedullary injections, as well as intrathecal, direct intraventricular, intraperitoneal, intranasal, or intraocular injections. Nevertheless, a preferred route of administration is the oral route.

The medicament or the pharmaceutical composition of the invention is, preferably, administered in a therapeutically effective dose. A therapeutically effective dose refers to an amount of the bacteriophage to be used in the composition applied, such that it prevents, ameliorates or treats the symptoms accompanying an infection of *Listeria* spp. in a subject.

The composition of the invention may be used to identify *Listeria* spp. present on (or within) foodstuffs, as well as those *Listeria* spp. bacteria present in the equipment or the general environment of the food processing plants in which the foodstuffs are being processed or in containers used for storage of foodstuffs and in animals infected with *Listeria* spp.

Thus, in another aspect, the present invention relates to an *in vitro* method for detecting the presence of a bacterium of *Listeria* spp., hereinafter "method of the invention", comprising
(i) incubating an isolated sample with the composition of the invention, wherein the bacteriophage of the composition of the invention has been genetically modified to incorporate a marker gene, and
(ii) detecting any change in said sample caused by the composition as an indication of the presence of *Listeria* spp.

The method of the invention allows the detection in a sample of any bacteria of *Listeria* spp. Nevertheless, in a particular embodiment, the bacteria is *L. monocytogenes, L. innocua, L. ivanovii, L. seeligeri,* or *L. welshimeri.* In a more particular embodiment, *L. monocytogenes* is *L. monocytogenes* serotype 1/2a, 1/2b, 1/2c, 3a, 3b, 3c, 4a, 4b, 4c, 4d or 4e.

In a first step, the method of the invention comprises incubating an isolated sample with the composition of the invention, wherein the bacteriophage of the composition of the invention has been genetically modified to incorporate a marker gene.

As use herein, the term "sample" refers to a small part or quantity of something intended to show what the whole is like. Thus, in a particular embodiment of the method of the invention, alone or in combination with all or each one of the previous particular embodiments, a food- or feed-product, a pharmaceutical, veterinary or biotechnological product, a water system, a fluid, a soil or a non-food surface. Additional explanations, examples and particulars about the different kinds of samples can be found herein in previous paragraphs, and they are applicable to the present inventive aspect. It is common general knowledge how to obtain these kinds of samples for their analysis.

In the method of the invention, the bacteriophage of the composition of the invention is genetically modified to incorporate a genetic system comprising DNA which encodes the expression of one or more detectable proteins (markers) which are not a gene product of *Listeria* spp. Thus, as the skilled person understands, the bacteriophage of the composition of the invention is used as a DNA recombinant vector specific for *Listeria* spp.

In this way, the DNA vector infects the bacteria of *Listeria* spp. and transfers the genetic system to the bacteria. The detectable proteins are expressed by the bacteria and the detection of the detectable proteins indicates the presence of *Listeria* spp. bacteria.

Thus, for detection of the presence of *Listeria* spp. bacteria, marker genes are employed. These are marker genes can be detected upon infection by the vector of a suitable host cell (in the present invention *Listeria* spp.) and subsequent culturing of the cells under conditions suitable for expression of the said marker genes. It is preferred that the marker genes are those which do not occur in the bacteria of *Listeria* spp., and which are inserted into the vector using recombinant techniques. Marker genes are widely known in the state of the art and any of them can be used in the method of the invention. They include bioluminescent proteins such as the lux gene which occurs in variants in various luminescent bacteria, for example of the genus *Vibrio.* The incorporation of the lux gene allows detection by luminescence measurement. An example of the lux gene is gene luxAB from *Vibrio harveyi.* Other suitable proteins include but are not limited to luciferase and fluorescent proteins such as green fluorescent protein.

After step (ii), the method of the invention comprises detecting any change in the sample caused by the composition as an indication of the presence of *Listeria* spp. Thus, in another particular embodiment, alone or in combination with all or each one of the previous particular embodiments, the change in the sample is due to lysis by the composition or a detectable label or signal.

The detection reaction can take place on a solid surface including but not limited to a test strip. In this embodiment, the recombinant bacteriophage (vector) containing the marker gene could be reversibly immobilized in or downstream from a sample application zone. Alternatively, the vector could be incubated with the sample prior to application on the test strip. *Anti-Listeria* antibodies would be irreversibly immobilized downstream from the vector and the sample application zone. If a sample is applied which contains *Listeria* spp., the vector would infect the *Listeria* spp. and the detectable proteins would be expressed. As the sample moves down the test strip, the *Listeria* spp. would become immobilized by the *anti-Listeria* antibodies. The marker proteins would then be detected in the immobilized *Listeria* spp.

### Isolated bacteriophages of the invention

In another aspect, the present invention relates to an isolated bacteriophage selected from the group consisting of bacteriophage AZT451 with deposit number CNCM I-5799, bacteriophage AZT356 with deposit number CNCM I-5797, bacteriophage AZT154 with deposit number CNCM I-5796, bacteriophage AZT450 with deposit number CNCM I-5798, bacteriophage AZT153 with deposit number CNCM I-5795, and bacteriophage AZT150 with deposit number CNCM I-5794. Herein, "isolated bacteriophages of the invention".

As used herein, "isolated" will mean material removed from its original environment (e.g., the natural environment in which the material occurs), and thus is "altered by the hand of man" from its natural environment. Thus, isolated material encompasses isolated *Listeria* spp. bacteriophages, isolated and cultured separately from the environment in which it was located. These isolates are present in purified compositions that do not contain any significant amount of other bacteriophage or bacterial strains, respectively.

The features of the isolated bacteriophages of the invention have been disclosed and explained above for the composition of the invention, and they are applicable to the present inventive aspect.

### DESCRIPTION OF THE DRAWINGS

**Figure 1****.** Electron micrographs of the six novel Listeria-bacteriophages: AZT451 (A), AZT356 (B), AZT154 (C), AZT450 (D), AZT153 (E) and AZT150 (F).
**Figure 2****.** Phylogenetic tree of the six novel bacteriophages herein described: AZT451, AZT356, AZT154, AZT450, AZT153, and AZT150 together with other described bacteriophages.
**Figure 3****.** Detail of the different types of lytic profiles indicating different bacteria susceptibility to phage infection. -: no lysis (resistant bacteria); +: opaque / semi-clear lysis (moderately susceptible bacteria); ++: clear lysis (highly susceptible bacteria).

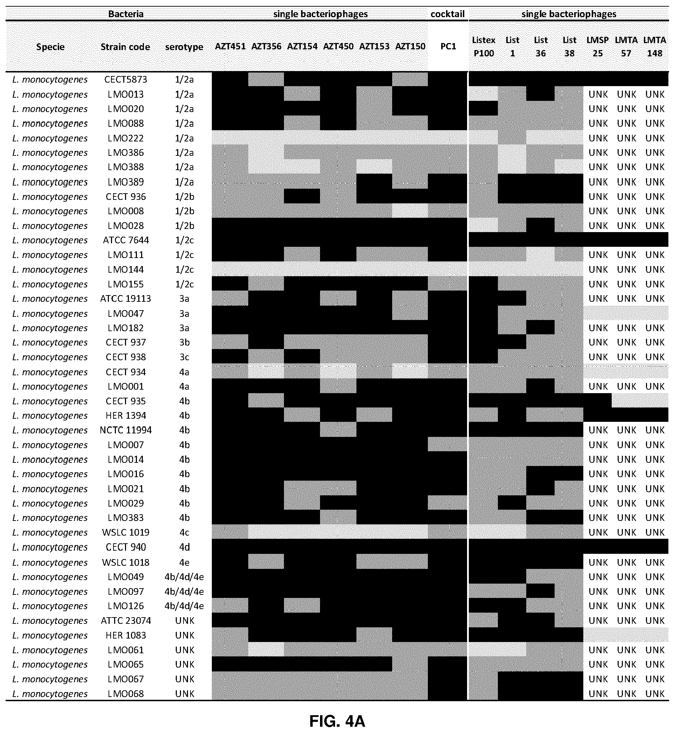

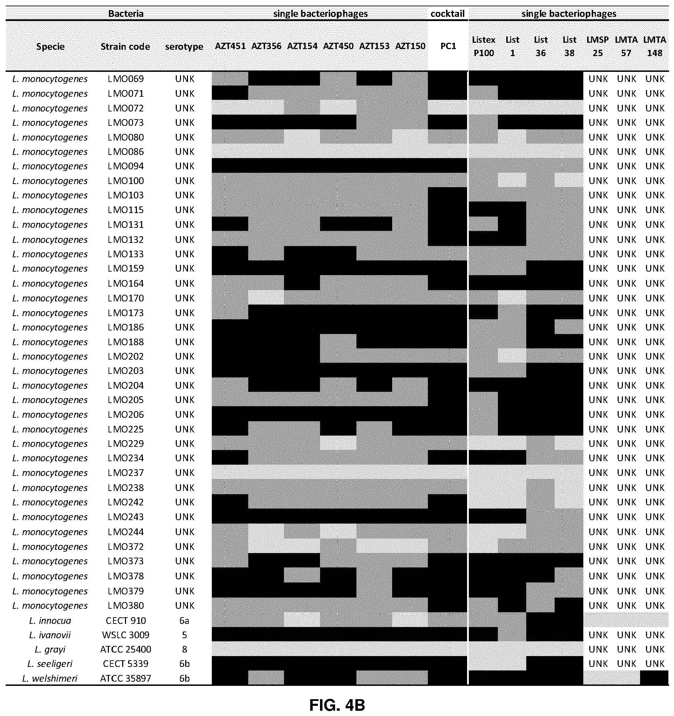

**Figure 4A** **and** **Figure 4B****.** Lytic specificity of the six novel *Listeria* bacteriophages and one phage cocktail (PC1) of the present invention as well as other *Listeria* bacteriophages onto different wild and/or collection strains of *L monocytogenes, L. innocua, L. ivanovii, L. grayi, L. seeligeri* and *L. welshimeri.* Data regarding the lytic specificity of the bacteriophages LMSP-25, LMTA-57, and LMTA-148 were taken from the patent US8685697 B1, whereas the other results were experimentally obtained. Results are shown in light gray, dark gray and black representing no lysis (resistant bacteria), opaque / semi-clear lysis (moderately susceptible bacteria) and clear lysis (highly susceptible bacteria), respectively.
**Figure 5****.** Lytic specificity of the six novel *Listeria* bacteriophages and one phage cocktail (PC1) of the present invention onto different *non-Listeria* strains. Results are shown in light gray, dark gray and black representing no lysis (resistant bacteria), opaque / semi-clear lysis (moderately susceptible bacteria), and clear lysis (susceptible bacteria), respectively.

### Examples

This invention refers to the novel and safe bacteriophages AZT451, AZT356, AZT154, AZT450, AZT153, and AZT150, deposited in the Collection Nationale de Cultures de Microorganismes (CNCM) of INSTITUT PASTEUR under the accession numbers CNCM I-5799, CNCM I-5797, CNCM I-5796, CNCM I-5798, CNCM I-5795 and CNCM I-5794, respectively. These bacteriophages are *Listeria* specific bacteriophages from the *Caudovirales* order belonging to the *Myoviridae* family, with an icosahedral capsid containing double stranded DNA and a contractile tail.

These novel bacteriophages, with large genomes of about 140 kb and a broad host range, are useful for the biological control of *L. monocytogenes* of different origins and serotypes as well as for the control of other *Listeria* species.

### Example 1. Bacterial strains and growth conditions

A total of 102 wild and collection bacterial strains were used to isolate and characterize the new isolated bacteriophages (**Table 1**). Specifically, 80 *L. monocytogenes* strains of diverse origin and serotypes, five strains of other *Listeria* species (*L. innocua, L. ivanovii, L. grayi, L. seeligeri* and *L. welshimeri*) and other 17 *non-Listeria* strains belonging to genus *Aeromonas, Bacillus, Campylobacter, Clostridium, Escherichia, Pseudomonas, Salmonella, Shigella, Staphylococcus* and *Vibrio,* were used to characterize the host range and *Listeria* specificity of isolated bacteriophages (**Table 1**).

**Table 1. Bacterial strains used to determine the lytic spectrum of the herein described Listeria bacteriophages. Available data on the serotype and origin of the strains are included.**

| **Strain** | **Strain code** | **Serotype** | **origin** |
|---|---|---|---|
| *L. monocytogenes* | CECT5873 (ATCC 35152) | 1/2a | pig |
| *L. monocytogenes* | LMO013 | 1/2a | raw cow milk |
| *L. monocytogenes* | LMO020 | 1/2a | smoked salmon |
| *L. monocytogenes* | LMO088 | 1/2a | fish |
| *L. monocytogenes* | LMO222 | 1/2a | smoked salmon |
| *L. monocytogenes* | LMO386 | 1/2a | liver dumplings |
| *L. monocytogenes* | LMO388 | 1/2a | herring fillets |
| *L. monocytogenes* | LMO389 | 1/2a | sausage |
| *L. monocytogenes* | CECT 936 | 1/2b | UNK |
| *L. monocytogenes* | LMO008 | 1/2b | raw cow milk |
| *L. monocytogenes* | LMO028 | 1/2b | sheep cheese |
| *L. monocytogenes* | ATCC 7644 | 1/2c | human |
| *L. monocytogenes* | LMO111 | 1/2c | pastry |
| *L. monocytogenes* | LMO144 | 1/2c | smoked salmon |
| *L. monocytogenes* | LMO155 | 1/2c | pork sausage |
| *L. monocytogenes* | ATCC19113 | 3a | human |
| *L. monocytogenes* | LMO047 | 3a | cold smoked salmon |
| *L. monocytogenes* | LMO182 | 3a | smoked salmon |
| *L. monocytogenes* | CECT 937 | 3b | 15-day old child |
| *L. monocytogenes* | CECT 938 | 3c | UNK |
| *L. monocytogenes* | CECT934 (ATCC 19114) | 4a | sheep brain |
| *L. monocytogenes* | LMO001 | 4a | raw cow milk |
| *L. monocytogenes* | CECT 935 (ATCC 13932) | 4b | child meningitis |
| *L. monocytogenes* | HER 1394 (ATCC19115) | 4b | UNK |
| *L. monocytogenes* | NCTC 11994 | 4b | human |
| *L. monocytogenes* | LMO007 | 4b | raw cow milk |
| *L. monocytogenes* | LMO014 | 4b | raw cow milk |
| *L. monocytogenes* | LMO016 | 4b | raw sheep milk |
| *L. monocytogenes* | LMO021 | 4b | smoked salmon |
| *L. monocytogenes* | LMO029 | 4b | cheese |
| *L. monocytogenes* | LMO383 | 4b | smoked salmon |
| *L. monocytogenes* | WSLC 1019 | 4c | UNK |
| *L. monocytogenes* | CECT 940 (ATCC 19117) | 4d | sheep |
| *L. monocytogenes* | WSLC 1018 | 4e | UNK |
| *L. monocytogenes* | LMO049 | 4b/4d/4e | salmon |
| *L. monocytogenes* | LMO097 | 4b/4d/4e | smoked salmon |
| *L. monocytogenes* | LMO126 | 4b/4d/4e | mussel |
| *L. monocytogenes* | ATCC 23074 | 4b | UNK |
| *L. monocytogenes* | HER 1083 (ATCC19118) | 4e | chicken |
| *L. monocytogenes* | LMO061 | UNK | pork sausage |
| *L. monocytogenes* | LMO065 | UNK | pizza |
| *L. monocytogenes* | LMO067 | UNK | sandwich |
| *L. monocytogenes* | LMO068 | UNK | sandwich |
| *L. monocytogenes* | LMO069 | UNK | soft cheese |
| *L. monocytogenes* | LMO071 | UNK | sausage |
| *L. monocytogenes* | LMO072 | UNK | smoked salmon |
| *L. monocytogenes* | LMO073 | UNK | cooked ham |
| *L. monocytogenes* | LMO080 | UNK | smoked salmon |
| *L. monocytogenes* | LMO086 | UNK | smoked bacon |
| *L. monocytogenes* | LMO094 | UNK | soft cheese |
| *L. monocytogenes* | LMO100 | UNK | cheese burguer |
| *L. monocytogenes* | LMO103 | UNK | cooked ham |
| *L. monocytogenes* | LMO115 | UNK | pate |
| *L. monocytogenes* | LMO131 | UNK | cooked ham |
| *L. monocytogenes* | LMO132 | UNK | smoked bacon |
| *L. monocytogenes* | LMO133 | UNK | cured bacon |
| *L. monocytogenes* | LMO159 | UNK | pig head sausage |
| *L. monocytogenes* | LMO164 | UNK | pate |
| *L. monocytogenes* | LMO170 | UNK | soft cheese |
| *L. monocytogenes* | LMO173 | UNK | green pepper |
| *L. monocytogenes* | LMO186 | UNK | foie |
| *L. monocytogenes* | LMO188 | UNK | soft cheese |
| *L. monocytogenes* | LMO202 | UNK | cheese |
| *L. monocytogenes* | LMO203 | UNK | cabbage |
| *L. monocytogenes* | LMO204 | UNK | cabbage |
| *L. monocytogenes* | LMO205 | UNK | cut-fruit |
| *L. monocytogenes* | LMO206 | UNK | oatmeal |
| *L. monocytogenes* | LMO225 | UNK | cooked ham |
| *L. monocytogenes* | LMO229 | UNK | cooked ham |
| *L. monocytogenes* | LMO234 | UNK | cooked ham |
| *L. monocytogenes* | LMO237 | UNK | cooked ham |
| *L. monocytogenes* | LMO238 | UNK | cooked ham |
| *L. monocytogenes* | LMO242 | UNK | pate |
| *L. monocytogenes* | LMO243 | UNK | cooked ham |
| *L. monocytogenes* | LMO244 | UNK | pork sausage |
| *L. monocytogenes* | LMO372 | UNK | UNK |
| *L. monocytogenes* | LMO373 | UNK | UNK |
| *L. monocytogenes* | LMO378 | UNK | cheese |
| *L. monocytogenes* | LMO379 | UNK | cheese |
| *L. monocytogenes* | LMO380 | UNK | turkey breast |
| *L. innocua* | CECT910 (ATCC 33090) | 6a | cow brain |
| *L. ivanovii* | WSLC 3009 | 5 | UNK |
| *L.grayi* | CECT 4181 (ATCC 25400) | 8 | corn stalks & leaves |
| *L. seeligeri* | CECT 5339 | 6b | animal faeces |
| *L. welshimeri* | CECT919 (ATCC 35897) | 6b | vegetation |
| *Campylobacter jejuni subsp. Jejuni* | CECT 8170 | UNK | human diarrhea |
| *Campylobacter coli* | CECT8205 | UNK | porcine faeces |
| *Escherichia coli O157:H7* | CECT 4972 (ATCC 700728) | UNK | human diarrhea |
| *Escherichia coli* | CECT 5947 | UNK | human diarrhea |
| *Escherichia coli* | CECT 516 (ATCC 8739) | UNK | human faeces |
| *Salmonella enterica subsp. entérica* | CECT 4156 | UNK | chicken |
| *Salmonella enterica subsp. Enterica* | CECT 409 (ATCC 19430) | UNK | UNK |
| *Staphylococcus aureus* | CECT 435 | UNK | human |
| *Vibrio vulnificus* | CECT529 (ATCC 27562) | UNK | human blood |
| *Vibrio alginolyticus* | CECT521 (ATCC 17749) | UNK | horse mackerel |
| *Vibrio parahaemolyticus* | CECT511 (ATCC 17802) | O1:K1 | human |
| *Clostridium perfringens* | CECT 376 (ATCC 13124) | UNK | bovine |
| *Pseudomonas aeruginosa* | CECT 108 (ATCC 27853) | UNK | blood |
| *Bacillus cereus* | CECT 131 (ATCC 10876) | UNK | flask |
| *Bacillus subtilis subsp. Spizizenii* | CECT 39 (ATCC 6051) | UNK | blood |
| *Shigella flexneri* | CECT 8175 (ATCC 29903) | 2a | UNK |
| *Aeromonas caviae* | CECT 838 (ATCC 15468) | UNK | guinea pigs |

Most of these bacterial strains were isolated by AZTI (strain code LMO) from different food products and food processing environments. Other bacterial strains were also purchased to the CECT (Colección Española de Cultivos Tipo; Spanish Type Culture Collection), the ATCC (American Type Culture Collection), the WSLC (Weihenstephan *Listeria* collection, Germany), or the Félix d' Herelle Reference Center collection (Université Laval in Quebec City, Canada; strain code HER). All bacterial strains were grown in Brain Heart Infusion (BHI; Oxoid, CM1135) broth or agar plates and incubated under their optimal growth temperature for 20 hours.

### Example 2. Bacteriophages isolation

Bacteriophages AZT451, AZT356, AZT154, AZT450, AZT153 and AZT150 were isolated from samples of cow faeces (AZT451 and AZT154), sheep faeces (AZT150 and AZT153), livestock environments (AZT356) and cooked ham (AZT450) collected in the Basque Country (Spain) from 2015 to 2016.

For their isolation, 10 g of sample were mixed with Tryptic Soy Broth (TSB) and an enrichment step was done by inoculating a mixture of exponential phase cultures of *Listeria* spp. hosts (**Table 1**) to a final concentration of 10⁶ Colony Forming Units (CFU)/mL.

Bacteriophage presence was evaluated after 24 hours of incubation at 30 °C by spotting 10 µL of the samples onto lawns of each of *the L. monocytogenes* isolates. Plates were incubated overnight at 30 °C and then examined for phage lytic plaques presence. Detected bacteriophages were then recovered and purified (**Table 2**). Briefly, single plaques were removed from the overlay agar using a sterile 1 ml pipette tip and resuspended in 900 µL SM buffer (50 mM Tris-HCl [pH7.5], 0.1 M NaCl, 8 mM MgSO4 y 0.01% w/v gelatine). Then, phages were propagated by the double agar layer method. Isolated phage plaques were picked and plated twice more to ensure purity. Fresh and pure monophage lysates were conserved at 4 °C in sterile tubes and at -80 °C in SM buffer supplemented with 50% glycerol.

**Table 2 - The novel isolated six Listeria-specific bacteriophages with corresponding CNCM accession number.**

| **Identification Reference Bacteriophage** | **CNCM Accession Number** |
|---|---|
| AZT150 | CNCM I-5794 |
| AZT153 | CNCM I-5795 |
| AZT154 | CNCM I-5796 |
| AZT356 | CNCM I-5797 |
| AZT450 | CNCM I-5798 |
| AZT451 | CNCM I-5799 |

The novel six Listeria-specific bacteriophages were received by the Collection Nationale de Cultures de Microorganismes (CNCM) of Institut Pasteur on 16 December 2021 for deposition under the Budapest Treaty. The bacteriophages were accepted (**Table 2**), and the following documents are available for each of them:
(1) the receipt referred to in Rule 7.1 of the Regulations under the Treaty (PCT), and
(2) the first viability statement referred to in Rule 10.2.a) i) PCT.

### Example 3. Bacteriophages propagation (production)

For bacteriophage lysates preparation, each phage was propagated in a suitable propagation host (**Table 1**) by the double agar layer method. Briefly, 250 µL of exponential phase bacterial cultures were mixed with 250 µL phage stock suspension. Mixtures were incubated for 15 min at 30 °C and added individually to 4 mL molten Tryptic Soy Agar (TSA) soft agar (supplemented with 0.4% Bacteriological Agar) enriched with 10 mM CaCl₂ and 5 mM MgSO₄ and previously tempered at 50 °C. The mixture of soft agar plus bacteria and phage was immediately poured onto TSA hard agar plates and allowed to dry for 15 minutes before incubation at 30 °C for 24 hours. Phages were recovered from plates presenting confluent lysis by adding 5 mL of SM buffer and incubated at 4 °C for 24 hours with gentle shaking. SM buffer with phages was treated with 1% chloroform prior to centrifugation at 10,000 x g for 10 minutes. The supernatant was filtered through a 0.45 µm syringe filter and kept at 4 °C until use. Bacteriophages can also be frozen or spray-dried for storage, and/or can be encapsulated and/or stabilized with proteins, polysaccharides, lipids or mixtures thereof. Phage lysates titer was determined by spotting 20 µL of serially diluted suspensions onto TSA soft agar overlay plates. Plates were allowed to dry for 15 minutes and incubated at 30 °C for 24 hours before phage plaques counting.

### Example 4. Bacteriophages stability under different temperature and pH conditions

The effect of temperature on the stability and infectivity of bacteriophages AZT451, AZT356, AZT154, AZT450, AZT153 and AZT150 was investigated at 4 °C and 25 °C in SM buffer (50 mM Tris-HCl, 0,1 M NaCl, 8 mM MgSOa y 0.01% w/v gelatin) at pH 7.5 for up to one year.

The effect of pH on the stability and infectivity of the bacteriophages was investigated at 4 ºC in SM at pH adjusted to values ranging from 2.0 to 9.5 (2.0, 3.5, 5.5, 7.5 and 9.5). Aliquots of each sample were taken at different times and were serially diluted to determine the phage titers by spotting dilutions onto TSA bacterial lawns containing the corresponding host strain. Plates were allowed to dry for 15 minutes and incubated at 30 °C for 24 hours before phage plaques counting.

All phages were stable at high titers and effective under storage at 4 °C (pH 7.5) for one year. The percentage of infection was more than 95% over the studied period **(Table 3A and Table 3B**), indicating the high stability of the novel bacteriophages over long time periods.

**Table 3A**

| Phage | | Time of storage at 4 °C (months) | | | |
|---|---|---|---|---|---|
| | | 0 | 1 | 3 | 4 |
| AZT451 | PFU/mL | 1.3x10⁹ | 1.2x10⁹ | 1.3x10⁹ | 1.2x10⁹ |
| | Infect. % | 100 | 90.0 | 97.5 | 90.0 |
| AZT356 | PFU/mL | 1.1x10⁹ | 1.0x10⁹ | 1.0x10⁹ | 1.0x10⁹ |
| | Infect. % | 100 | 97.8 | 95.2 | 95.2 |
| AZT154 | PFU/mL | 1.0x10⁹ | 9.8x10⁸ | 9.9x10⁸ | 1.0x10⁹ |
| | Infect. % | 100 | 94.8 | 96.1 | 96.8 |
| AZT450 | PFU/mL | 5.7x10⁹ | 5.6x10⁹ | 5.6x10⁹ | 5.6x10⁹ |
| | Infect. % | 100 | 98.2 | 98.2 | 98.2 |
| AZT153 | PFU/mL | 1.0x10⁹ | 9.8x10⁸ | 9.9x10⁸ | 9.6x10⁸ |
| | Infect. % | 100 | 98.0 | 99.3 | 96.0 |
| AZT150 | PFU/mL | 1.1x10⁸ | 1.1x10⁸ | 1.1x10⁸ | 1.1x10⁸ |
| | Infect. % | 100 | 98.5 | 98.2 | 98.5 |

**Table 3B**

| Phage | | Time of storage at 4 °C (months) | | |
|---|---|---|---|---|
| | | 6 | 9 | 12 |
| AZT451 | PFU/mL | 1.3x10⁹ | 1.3x10⁹ | 1.3x10⁹ |
| | Infect. % | 97.5 | 97.5 | 97.5 |
| AZT356 | PFU/mL | 1.0x10⁹ | 1.0x10⁹ | 1.0x10⁹ |
| | Infect. % | 95.2 | 95.2 | 95.2 |
| AZT154 | PFU/mL | 1.0x10⁹ | 1.0x10⁹ | 9.9x10⁸ |
| | Infect. % | 96.8 | 98.7 | 96.1 |
| AZT450 | PFU/mL | 5.5x10⁹ | 5.7x10⁹ | 5.7x10⁹ |
| | Infect. % | 95.9 | 99.4 | 99.4 |
| AZT153 | PFU/mL | 9.6x10⁸ | 9.7x10⁸ | 9.9x10⁸ |
| | Infect. % | 96.0 | 96.7 | 98.7 |
| AZT150 | PFU/mL | 1.1x10⁸ | 1.1x10⁸ | 1.1x10⁸ |
| | Infect. % | 98.5 | 95.5 | 95.5 |

**Table 3A and Table 3B.** Stability of the six novel Listeria-bacteriophages stored at 4 °C for one year. Table shows both the titer (PFU/mL) and the infectivity percentage of each bacteriophage at different time intervals of storage at 4 °C.

Furthermore, all the six bacteriophages presented a very good stability during their storage at 25 ºC (**Table 4**).

**Table 4. Stability of the six novel Listeria bacteriophages stored at 25 °C for four months. Table shows both the titer (PFU/mL) and the infectivity percentage of each bacteriophage at different time intervals of storage at 25 °C.**

| Phage | | Time of storage at 25 °C (months) | | | |
|---|---|---|---|---|---|
| | | 0 | 1 | 3 | 4 |
| AZT451 | PFU/mL | 2.2x10⁹ | 2.2x10⁹ | 1.8x10⁹ | 9.8x10⁸ |
| | Infect. % | 100 | 98.5 | 80.6 | 43.9 |
| AZT356 | PFU/mL | 1.7x10⁹ | 1.7x10⁹ | 1.4x10⁹ | 1.2x10⁹ |
| | Infect. % | 100 | 98.1 | 80.8 | 69.2 |
| AZT154 | PFU/mL | 2.5x10⁹ | 2.0x10⁹ | 1.2x10⁹ | 1.0x10⁹ |
| | Infect. % | 100 | 80 | 48 | 40 |
| AZT450 | PFU/mL | 1.0x10⁹ | 1.0x10⁹ | 1.0x10⁹ | 9.0x10⁸ |
| | Infect. % | 100 | 96.8 | 96.8 | 87.1 |
| AZT153 | PFU/mL | 1.2x10⁹ | 1.0x10⁹ | 1.8x10⁸ | 1.1x10⁸ |
| | Infect. % | 100 | 81.1 | 14.6 | 8.9 |
| AZT150 | PFU/mL | 1.2x10⁸ | 1.1x10⁸ | 1.1x10⁸ | 1.1x10⁸ |
| | Infect. % | 100 | 91.7 | 91.7 | 91.7 |

Phages AZT451, AZT356, AZT450 and AZT150 maintained more than 90% of the infectivity during the first month, while phages AZT153 and AZT154 slightly reduced the infectivity to 81.1 and 80%, respectively, over this period of time. However, slight reductions of 1 log maximum were observed in the titer of the six novel phages herein described after 4 months of storage, so all phages maintained stable and effective under storage at 25 ºC (pH 7.5) over this period of time.

Regarding their stability under different pH conditions (**Table 5**), all bacteriophages maintained stable at high titers and effective (percentage of infectivity greater than 90%) for 30 days at pH ranging from 3.5 to 9.5, except for the following exceptions. On the one hand, AZT451, AZT154 and AZT153 phages showed a slightly reduced percentage of infectivity of 84.6, 80 and 66.7%, respectively, at pH 3.5. And on the other hand, AZT450 and AZT154 phages exhibited a percentage of infectivity of 52.6 and 9.76%, respectively, at pH 5.5. Overall, the stability of all bacteriophages significantly decreased at pH 2.0 after 30 days of storage. AZT356 appeared as the most stable phage, with 90.9% of infectivity after 30 days at pH 2.0, followed by AZT154 (80.0%) and AZT451 (76.9%), whereas AZT153 (55.6%), AZT450 (15.8%) and AZT150 (9.5%) were the most acid-sensitive phages.

**Table 5. Stability of the six novel Listeria-bacteriophages at pH values ranging from 2.0 to 9.5 over 60 days of storage at 4 °C. Table shows both the titer (PFU/mL) and the infectivity percentage of each bacteriophage at different time intervals of storage.**

| Phage | pH | | Time of storage (days) | | |
|---|---|---|---|---|---|
| | | | 1 | 30 | 60 |
| AZT451 | 2.0 | PFU/mL | 1.3x10⁹ | 1.0x10⁹ | 8.0x10⁸ |
| | | Infect. % | 100 | 76.9 | 61.5 |
| | 3.5 | PFU/mL | 1.3x10⁹ | 1.1x10⁹ | 1.0x10⁹ |
| | | Infect. % | 100 | 84.6 | 76.9 |
| | 5.5 | PFU/mL | 1.3x10⁹ | 1.2x10⁹ | 1.3x10⁹ |
| | | Infect. % | 100 | 90.0 | 97.5 |
| | 7.5 | PFU/mL | 1.3x10⁹ | 1.2x10⁹ | 1.3x10⁹ |
| | | Infect. % | 100 | 90.0 | 97.5 |
| | 9.5 | PFU/mL | 1.3x10⁹ | 1.2x10⁹ | 1.2x10⁹ |
| | | Infect. % | 100 | 90.0 | 90.0 |
| AZT356 | 2.0 | PFU/mL | 1.0x10⁹ | 1.0x10⁹ | 7.0x10⁸ |
| | | Infect. % | 100 | 90.9 | 63.6 |
| | 3.5 | PFU/mL | 1.1x10⁹ | 1.0x10⁹ | 9.0x10⁸ |
| | | Infect. % | 100 | 90.9 | 81.8 |
| | 5.5 | PFU/mL | 1.1x10⁹ | 1.0x10⁹ | 1.0x10⁹ |
| | | Infect. % | 100 | 95.2 | 95.2 |
| | 7.5 | PFU/mL | 1.1x10⁹ | 1.0x10⁹ | 1.0x10⁹ |
| | | Infect. % | 100 | 97.8 | 95.2 |
| | 9.5 | PFU/mL | 1.1x10⁹ | 1.0x10⁹ | 9.8x10⁸ |
| | | Infect. % | 100 | 95.2 | 93.3 |
| AZT154 | 2.0 | PFU/mL | 1.0x10⁹ | 8.0x10⁸ | 1.0x10⁸ |
| | | Infect. % | 100 | 80.0 | 10.0 |
| | 3.5 | PFU/mL | 1.0x10⁹ | 8.0x10⁸ | 5.0x10⁸ |
| | | Infect. % | 100 | 80.0 | 50.0 |
| | 5.5 | PFU/mL | 1.0x10⁹ | 1.0x10⁸ | 6.0x10⁸ |
| | | Infect. % | 100 | 9.7 | 58.1 |
| | 7.5 | PFU/mL | 1.0x10⁹ | 9.8x10⁸ | 9.9x10⁸ |
| | | Infect. % | 100 | 94.8 | 96.1 |
| | 9.5 | PFU/mL | 1.0x10⁹ | 1.0x10⁹ | 9.8x10⁸ |
| | | Infect. % | 100 | 96.8 | 94.8 |
| AZT450 | 2.0 | PFU/mL | 5.7x10⁹ | 9.0x10⁸ | 2.0x10⁸ |
| | | Infect. % | 100 | 15.8 | 3.5 |
| | 3.5 | PFU/mL | 5.7x10⁹ | 1.0x10⁹ | 5.0x10⁸ |
| | | Infect. % | 100 | 17.5 | 8.8 |
| | 5.5 | PFU/mL | 5.7x10⁹ | 3.0x10⁹ | 3.0x10⁹ |
| | | Infect. % | 100 | 52.6 | 52.6 |
| | 7.5 | PFU/mL | 5.7x10⁹ | 5.6x10⁹ | 5.6x10⁹ |
| | | Infect. % | 100 | 98.2 | 98.2 |
| | 9.5 | PFU/mL | 5.7x10⁹ | 5.5x10⁹ | 1.2x10⁹ |
| | | Infect. % | 100 | 96.5 | 21.1 |
| AZT153 | 2.0 | PFU/mL | 9.0x10⁸ | 5.0x10⁸ | 9.0x10⁷ |
| | | Infect. % | 100 | 55.6 | 10.0 |
| | 3.5 | PFU/mL | 9.0x10⁸ | 6.0x10⁸ | 1.0x10⁸ |
| | | Infect. % | 100 | 66.7 | 11.1 |
| | 5.5 | PFU/mL | 2.2x10⁹ | 2.0x10⁹ | 2.1x10⁹ |
| | | Infect. % | 100 | 90.9 | 95.5 |
| | 7.5 | PFU/mL | 1.0x10⁹ | 9.8x10⁸ | 9.9x10⁸ |
| | | Infect. % | 100 | 98.0 | 99.3 |
| | 9.5 | PFU/mL | 1.0x10⁹ | 9.9x10⁸ | 8.0x10⁸ |
| | | Infect. % | 100 | 99.0 | 80.0 |
| AZT150 | 2.0 | PFU/mL | 1.1x10⁸ | 1.0x10⁷ | 8.0x10⁶ |
| | | Infect. % | 100 | 9.5 | 7.6 |
| | 3.5 | PFU/mL | 1.1x10⁸ | 1.0x10⁸ | 1.0x10⁶ |
| | | Infect. % | 100 | 95.2 | 95.2 |
| | 5.5 | PFU/mL | 1.1x10⁸ | 1.0x10⁸ | 1.0x10⁶ |
| | | Infect. % | 100 | 89.6 | 89.6 |
| | 7.5 | PFU/mL | 1.1x10⁸ | 1.1×10⁸ | 1.1x10⁸ |
| | | Infect. % | 100 | 98.5 | 98.2 |
| | 9.5 | PFU/mL | 1.1x10⁸ | 1.1x10⁸ | 1.0x10⁸ |
| | | Infect. % | 100 | 98.5 | 89.6 |

These results demonstrated that bacteriophages of the invention can be easily maintained in a simple carrier, such as SM, during a long period of time at 4 °C. Moreover, this carrier could be used as vehicle for administration purpose of the invention. The high stability of bacteriophages to tested temperatures and pH values demonstrated that bacteriophages of the invention could be stable at high titers and effective under storage and application conditions of temperature and pH, and then used for the purposes of the invention.

### Example 5. Bacteriophages electron microscopy.

Bacteriophages AZT451, AZT356, AZT154, AZT450, AZT153 and AZT150 at high titers of 10⁸ - 10⁹ PFU/mL were applied to electron carbon coated grids and negatively stained with 2% uranyl acetate by CNB (Centro Nacional de Biotecnología - CSIC, Spain). After drying, preparations were examined with a transmission electron microscope at different magnitudes. Phage morphology and dimensions were recorded.

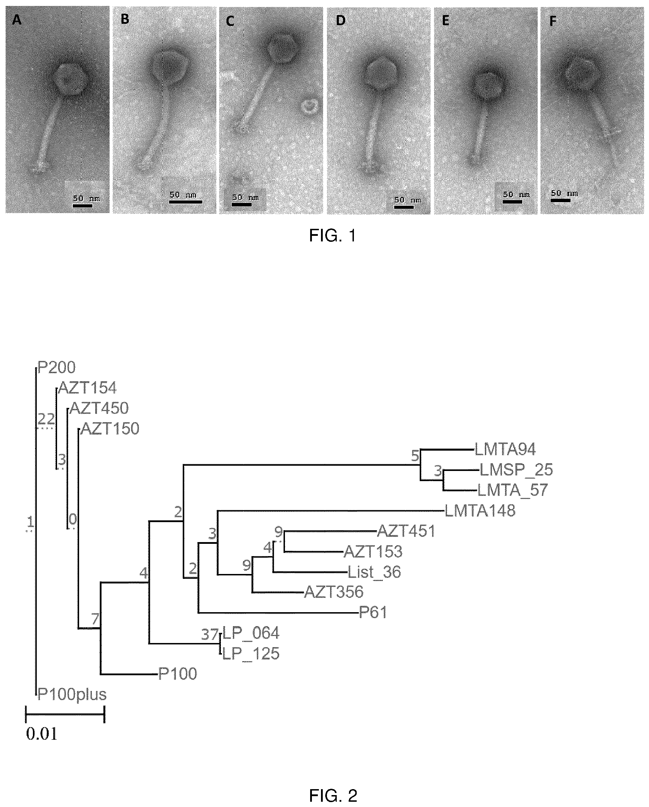

Electron micrographs of negatively stained bacteriophages revealed icosahedral heads and long contractile tails (**Figure 1** and **Table 6**). Therefore, attending to their morphology, all bacteriophages belong to the *Myoviridae* family of the *Caudovirales* order.

**Table 6. Head and Tail dimensions of the six novel Listeria bacteriophages. Between ten and forty bacteriophage particles were measured in the obtained TEM images by using imaged software (Abramoff, M.D., et al. Biophotonics International, volume 11, issue 7, pp. 36-42, 2004). Table shows the calculated mean value and the Standard Deviation (SD) expressed in nm.**

| Phage | Tail length Mean ± SD (nm) | Head diameter Mean ± SD (nm) |
|---|---|---|
| AZT451 | 204.4 ± 14.1 | 93.0 ± 6.4 |
| AZT356 | 200.0 ± 9.5 | 90.4 ± 6.4 |
| AZT154 | 199.0 ± 14.2 | 83.3 ± 5.9 |
| AZT450 | 208.5 ± 11.5 | 92.4 ± 5.8 |
| AZT153 | 190.7 ± 20.6 | 89.4 ± 6.2 |
| AZT150 | 211.3 ± 17.4 | 94.5 ± 6.3 |

### Example 6. Bacteriophages genome characterization

DNA of bacteriophages AZT451, AZT356, AZT154, AZT450, AZT153 and AZT150 was isolated using a suitable commercial kit (QIAamp DNA kit, Qiagen Inc.) and sent to Microsynth (Switzerland) to be sequenced. Sequence quality analysis of the obtained reads was performed by FastQC (Reference: Andrews, S. (2010). FastQC: A Quality Control Tool for High Throughput Sequence Data [Online]. Available online at: http://www.bioinformatics.babraham.ac.uk/projects/fastqc/) After filtering, DNA assembly of the reads was performed by means of SPAdes software (Bankevich et al 2012. J Comput Biol.;19(5):455-77). The quality of the obtained assemblies was evaluated using QUAST (Gurevich et al 2013, Bioinformatics. 2013;29(8):1072-1075). Based on the DNA sequencing data, the genome size of bacteriophages AZT451, AZT356, AZT154, AZT450, AZT153 and AZT150 was 134,964 bp, 135,194 bp, 135,025 bp, 135,022 bp, 136,556 bp and 136,055 bp, respectively. The study of DNA sequence of these bacteriophages showed that all of them were virulent (lytic) bacteriophages suitable for practical applications and allowed to classify them within the genus P100-like, according to the rules for the classification of bacteriophages (Adriaenssens and Rodney Brister 2017, Viruses, 9(4):70). These data are in agreement with those obtained by electron microscopy regarding morphological characterization (Example 5). Furthermore, the analysis of DNA sequences showed that no virulence or antibiotic resistance genes are encoded in the genomes of these bacteriophages. Results are summarized in **Table 7A** and **Table 7B**.

**Table 7A**

| | **AZT 451** | **AZT 356** | **AZT 154** |
|---|---|---|---|
| **Genome (bp)** | 134964 | 135194 | 135025 |
| **GC content (%)** | 35,9 | 35,9 | 35,9 |
| **Genus** | P100-like | P100-like | P100-like |
| **Family** | *Myoviridae* | *Myoviridae* | *Myoviridae* |
| **Order** | *Caudovirales* | *Caudovirales* | *Caudovirales* |
| **Toxin/Virulence genes** | None | None | None |
| **Lysogeny genes** | None | None | None |
| **Abx-Resistance genes** | None | None | None |

**Table 7B**

| | **AZT 450** | **AZT 153** | **AZT 150** |
|---|---|---|---|
| **Genome (bp)** | 135022 | 136556 | 135055 |
| **GC content (%)** | 35,9 | 35,9 | 35,9 |
| **Genus** | P100-like | P100-like | P100-like |
| **Family** | *Myoviridae* | *Myoviridae* | *Myoviridae* |
| **Order** | *Caudovirales* | *Caudovirales* | *Caudovirales* |
| **Toxin/Virulence genes** | None | None | None |
| **Lysogeny genes** | None | None | None |
| **Abx-Resistance genes** | None | None | None |

**Table 7A and Table 7B**. Summary data of the herein described six novel bacteriophages.

Obtained DNA sequences were compared among them and with other known bacteriophages using NCBI BLAST (Altschul et al. 1990, J. Mol. Biol. 215:403-410). Results are summarized in **Table 8A** and **Table 8B.** The obtained comparison results evidence significant differences at genome level among the six novel bacteriophages of the present invention and the analyzed bacteriophages known in the state of the art.

**Table 8A.**

| | **P100** | **List-36** | **LMSP-25** | **LMTA-57** | **LMTA-94** | **LMTA-148** |
|---|---|---|---|---|---|---|
| **AZT451** | QC:96% | QC:97% | QC:97% | QC:97% | QC:97% | QC:96% |
| | PI:98.07% | PI:99.95% | Pl:97.13% | PI:97.06% | PI:97.33% | PI:98.34% |
| **AZT356** | QC:97% | QC:97% | QC:96% | QC:96% | QC:96% | QC:95% |
| | PI:98.79% | PI:98.77% | PI:97.11% | PI:97.06% | PI:97.15% | PI:98.31% |
| **AZT154** | QC:97% | QC:95% | QC:97% | QC:97% | QC:96% | QC:95% |
| | PI:99.96% | PI:97.56% | PI:96.65% | PI:96.63% | PI:96.61% | PI:98.20% |
| **AZT450** | QC:97% | QC:95% | QC:97% | QC:97% | QC:96% | QC:95% |
| | PI:99.98% | PI:97.80% | PI:97.28% | PI:97.21% | PI:97.21% | PI:98.34% |
| **AZT153** | QC:95% | QC:96% | QC:96% | QC:95% | QC:96% | QC:94% |
| | PI:98.33% | PI:99.82% | PI:97.17% | PI:97.09% | PI:97.36% | PI:98.24% |
| **AZT150** | QC:97% | QC:95% | QC:97% | QC:97% | QC:96% | QC:95% |
| | PI:99.99% | PI:97.79% | PI:97.28% | PI:97.20% | PI:97.21% | PI:98.33% |

**Table 8B.**

| | **LP-125** | **LP-064** | **P200** | **P100plus** | **P61** |
|---|---|---|---|---|---|
| **AZT451** | QC:98% | QC:98% | QC:98% | QC:98% | QC:98% |
| | PI:.96% | PI:97.96% | PI:98.08% | PI:98.07% | PI:97.60% |
| **AZT356** | QC:98% | QC:98% | QC:98% | QC:98% | QC:98% |
| | PI:98.13% | PI:98.16% | PI:98.79% | Pl:98.78% | PI:97.52% |
| **AZT154** | QC:99% | QC:99% | QC:100% | QC:100% | QC:97% |
| | PI:98.44% | PI:98.44% | PI:99.98% | PI:99.98% | PI:97.45% |
| **AZT450** | QC:99% | QC:99% | QC:100% | QC:100% | QC:97% |
| | PI:98.31% | PI:98.30% | PI:99.99% | PI:99.98% | PI:97.62% |
| **AZT153** | QC:97% | QC:97% | QC:98% | QC:98% | QC:96% |
| | PI:98.08% | PI:98.08% | PI:98.33% | PI:98.33% | PI:97.61% |
| **AZT150** | QC:99% | QC:99% | QC:100% | QC:100% | QC:97% |
| | Pl:98.35% | PI:98.34% | PI:99.99% | PI:99.99% | PI:97.62% |

**Table 8A and Table 8B.** NCBI BLAST comparisons of the six novel bacteriophages with other relevant *Listeria* bacteriophages described elsewhere. (QC: Query cover; PI: percentage of identity).

Bioinformatic analyses of the newly sequenced bacteriophages allowed the construction of a phylogenetic tree using VICTOR (Jan P Meier-Kolthoff and Markus Göker, 2017. Bioinformatics, Volume 33, Issue 21, 01 November 2017, Pages 3396-3404, https://doi.org/10.1093/bioinformatics/btx440), and Tree view (Page RDM., 1996. Bioinformatics, Volume 12, Issue 4, Pages 357-358) for visualization,http://etetoolkit.org/treeview/ as shown in **Figure 2****.** Previously evidenced genome differences among analysed bacteriophages are also depicted in the phylogenetic tree built from sequences.

### Example 7. Bacteriophages lytic specificity characterization

One hundred and two bacterial strains, including 80 *L. monocytogenes,* 1 *L. innocua,* 1 *L. ivanovii,* 1 *L. seeligeri,* 1 *L. grayi,1 L. welshimeri,* and 17 other *non-Listeria* strains (**Table 1**) were screened for their susceptibility to the deposited bacteriophages. Moreover, several different combinations of the novel bacteriophages were also tested, since it is the main accepted strategy to obtain a broader specificity range against target *Listeria* strains and limit the potential emergence of bacteriophageresistance. Additionally, bacterial susceptibility to other *Listeria* specific bacteriophages, such as P100 (PhageGuard Listex^{™} P100 acquired to Betelgeux-Spain) or List-1, List-36 and List-38 (provided by ATCC under codes ATCC-PTA-5372, ATCC-PTA-5376 and ATCC-PTA-5377, respectively), was also screened.

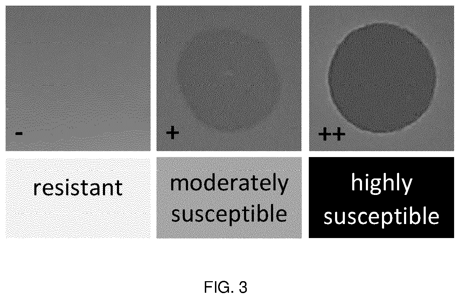

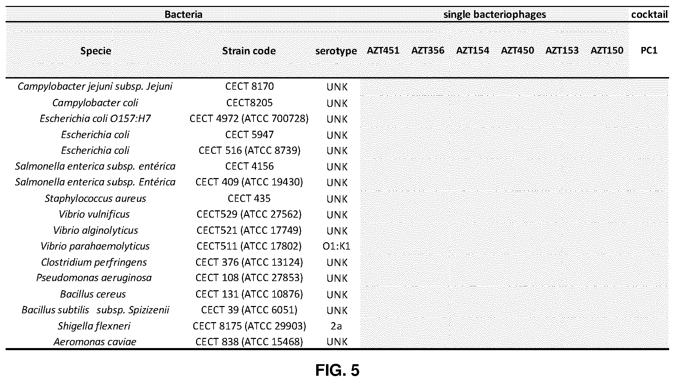

The spot test method was used for the lytic specificity characterization of each single monophage or multiple bacteriophage combinations. Very briefly, 10 µL of each bacteriophage suspensions (10⁹ PFU/mL) were spotted onto lawns of the 102 bacterial strains in TSA agar plates. After allowing the plates to dry for 20 minutes at room temperature, they were incubated at optimum temperature of each bacterium for 24-48 hours. The appearance of confluent lysis plaques was considered a positive result. Results were classified as: -: no lysis, +: confluent semi-clear or opaque lysis, and ++: confluent clear lysis, allowing bacterial hosts to be identified as resistant, moderately susceptible or highly susceptible to bacteriophages, respectively, as indicated in **Figure 3****.** Lytic specificity results are presented in **Figures 4A****,** **4B** and **5****,** and **Tables 9.**

Results of **Figures 4A** **and** **4B** and **Table 9** revealed that the six novel bacteriophages of the present invention showed different lytic spectrum than the other 7 studied *Listeria* bacteriophages (P100, List-1, List-36, List-38, LMSP-25, LMTA-57 and LMTA-148) against the different strains of *Listeria* spp. tested. These results demonstrated that the six novel bacteriophages of the present invention are clearly different among them and from the other 7 studied ones. Regarding their anti*-Listeria* effectiveness, bacteriophage AZT451 exhibited the broadest host range or lytic spectrum (total susceptible strains of 93%), as well as the best efficacy (highly susceptible *Listeria* strains of 55% with only 7% of non-susceptible (resistant) strains).

**Table 9. Summary data of the results presented in Figures 4A and 4B, regarding the lytic specificity of the six novel Listeria bacteriophages of the present invention as well as other Listeria bacteriophages onto different strains of Listeria spp.**

| Phage | *Listeria* st rains | | | | |
|---|---|---|---|---|---|
| | N | Resistant | Moderately susceptible | Highly susceptible | Total susceptible |
| | | n (%) | n (%) | n (%) | n (%) |
| **AZT451** | 85 | 6 (7%) | 32 (38%) | 47 (55%) | 79 (93%) |
| **AZT356** | 85 | 14 (16%) | 28 (33%) | 43 (51%) | 71 (84%) |
| **AZT154** | 85 | 10 (12%) | 32 (38%) | 43 (51%) | 75(88%) |
| **AZT450** | 85 | 10 (12%) | 35 (41%) | 40 (47%) | 75 (88%) |
| **AZT153** | 85 | 8 (9%) | 40 (47%) | 37 (44%) | 77(91%) |
| **AZT150** | 85 | 11 (13%) | 38 (45%) | 36 (42%) | 74 (87%) |
| **PC1** | 85 | 6 (7%) | 19 (22%) | 60 (71%) | 79 (93%) |
| **Listex P100** | 85 | 16 (19%) | 40 (47%) | 29 (34%) | 69 (81%) |
| **List-1** | 85 | 18 (21%) | 31 (36%) | 36 (42%) | 67 (79%) |
| **List-36** | 85 | 7 (8%) | 39 (46%) | 39 (46%) | 78 (92%) |
| **List-38** | 85 | 11 (13%) | 43 (51%) | 31 (36%) | 74 (87%) |
| **LMSP25** | 9 | 4 (44%) | UNK | UNK | 5 (56%) |
| **LMTA57** | 9 | 5 (56%) | UNK | UNK | 4 (44%) |
| **LMTA148** | 9 | 4 (44%) | UNK | UNK | 5 (56%) |

As shown in **Figure 4A****,** **Figure 4B** and **Table 9,** the six novel bacteriophages of the present invention and the bacteriophage cocktail PC1 (comprising the five phages AZT451, AZT154, AZT450, AZT153, AZT150) could infect between the 84% and the 93% of tested *Listeria* isolates, including *L. innocua, L. ivanovii, L. seeligeri, L. welshimeri* and *L. monocytogenes* serotypes 1/2a, 1/2b, 1/2c, 3a, 3b, 3c, 4a, 4b, 4d, and 4e. Both AZT451 and PC1 could also infect *L. monocytogenes* serotype 4c. Furthermore, all novel bacteriophages were capable of infecting various prophage-free *Listeria* strains, including the non-pathogenic prophage-free WSLC 3009 *L. ivanoviistrain* (Klumpp etal., 2014. Genome Announc. 2(2): e00404-14)); which could be safely used as propagation hosts for large industrial scale production.

AZT356 bacteriophage showed the narrowest host range (total susceptible strains of 84%), whereas bacteriophage AZT451 and cocktail PC1 infected up to 93% of tested *Listeria* strains. Furthermore, the combination of bacteriophages in the phage cocktail PC1, revealed a significant effect on the *Listeria* susceptibility to bacteriophage infection with an important increase in the number of highly susceptible strains after application of phage cocktail PC1. Specifically, between 42% and 55% of tested *Listeria* strains were highly susceptible to infection by any single bacteriophage of the present invention, whereas up to the 71% of these strains showed high susceptibility to phage cocktail PC1.

As expected, all novel bacteriophages and their bacteriophage cocktails were not capable of infecting any of the *non-Listeria* species strains (**Figure 5**), demonstrating their specificity for *Listeria* species.

### Example 8. In vitro killing assays.

The *in vitro* efficacy of the phage cocktail 1 (PC1 comprising the phages AZT451, AZT153, AZT450, AZT150, AZT154) to a dose of 10⁷ PFU/mL was evaluated against a mixture of two *L. monocytogenes* strains, *LMO020 and CECT4032,* of serotypes 1/2a and 4b, respectively, previously cultured at refrigeration conditions (8 °C) and inoculated at different counts of 10⁴, 10² and 50 CFU/mL, by monitoring *Listeria* counts for 35 days of storage under refrigerated conditions (**Table 10**). The results showed that phage addition induced a fast reduction in *L. monocytogenes* counts, with absence of *Listeria* growth in treated samples after 35 days of storage under refrigerated conditions.

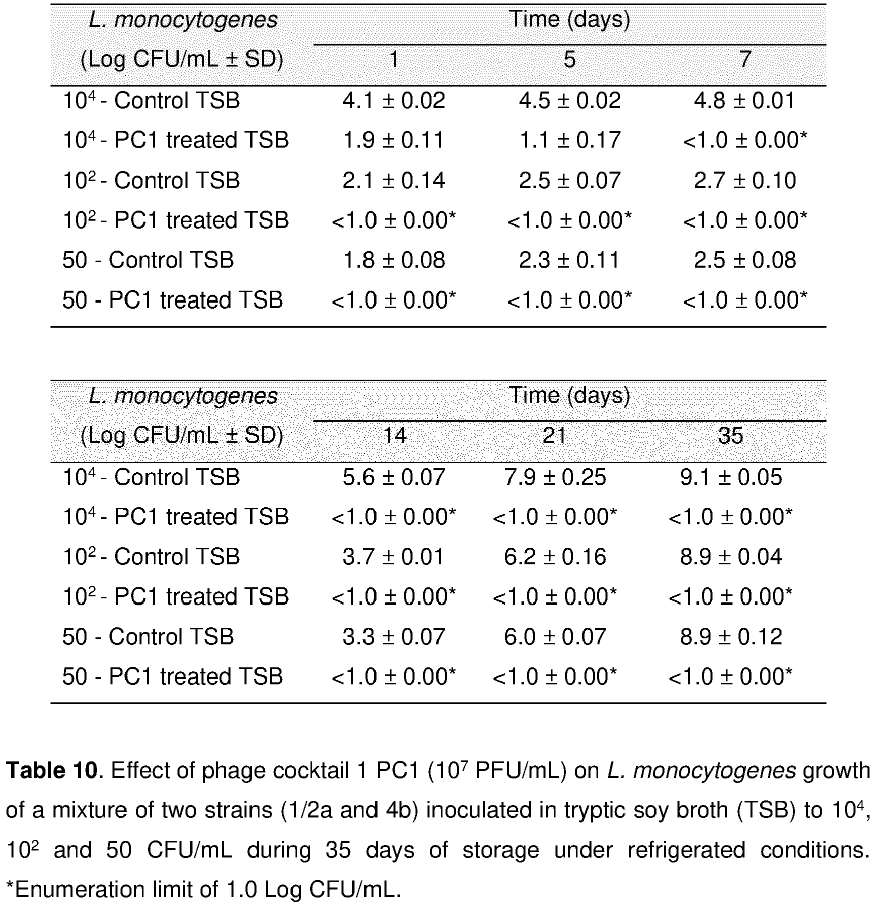

### Example 9. Application of bacteriophage cocktails/formulations to reduce Listeria from food products.

The efficacy of phage cocktail 1 (PC1) on *L. monocytogenes* growth during the shelf life of one raw (fresh salmon) and one ready to eat (RTE; cold smoked salmon) food product was evaluated. For this purpose, the fish products were inoculated with a twostrain mixture of *L. monocytogenes* (LMO020, ser. 1/2a; and CECT4032, ser. 4b), previously cultured at refrigeration conditions (8 °C) at naturally occurring low *L*. *monocytogenes* counts of 10² and 50 CFU/g, and surface treated by applying the bacteriophage formulation to a dose of 10⁷ PFU/g. After 30 minutes, samples were vacuum packed and stored under refrigeration conditions. For each type of nontreated (control) and treated fish product (fresh salmon and cold smoked salmon), at least 4 samplings with 3 analysed samples per sampling were performed over the shelf-life period. Samples were analysed by the ALOA count enumeration method for *L. monocytogenes,* alternative to the reference standard method EN ISO 11290-2 and previously validated by EN ISO 16140, being the enumeration limit of 10 CFU/g.

Results in raw salmon and cold smoked salmon contaminated at naturally occurring low *L. monocytogenes* counts of 50 and 100 CFU/g showed that bacteriophage cocktail, when applied at a dose of 10⁷ PFU/g and MOI (multiplicity of infection: ratio between bacteriophage dose and *L. monocytogenes* load) of 10⁵, is able to reduce the *L. monocytogenes* load in an appropriate way to avoid this load to exceed 100 CFU/g at the end of the shelf-life of these fish products (**Tables 11 and 12**). Therefore, the bacteriophages and/or cocktails/formulations of this invention are a valuable solution for reducing the load of *L. monocytogenes* pathogenic bacteria in food (and feed) products, and in food manufacturing processes.

**Table 11. Effect of phage cocktail 1 PC1 (10⁷ PFU/mL) on L. monocytogenes growth of a mixture of two strains (1/2a and 4b) inoculated to 10² and 50 CFU/g on the surface of raw salmon (RS) fillets during their shelf life under refrigerated conditions.**

| *L. monocytogenes* (Log CFU/mL ± SD) | Time (days) | | | |
|---|---|---|---|---|
| | 1 | 5 | 7 | 11 |
| 10² - Control RS | 2.3 ± 0.18 | 3.5 ± 0.97 | 4.6 ± 0.71 | 5.8 ± 0.71 |
| 10² - PC1 treated RS | <1.0 ± 0.00* | 1.2 ± 0.29 | 1.3 ± 0.37 | 1.8 ± 0.78 |
| 50 - Control RS | 1.8 ± 0.31 | 2.6 ± 0.57 | 3.7 ± 1.27 | 4.6 ± 1.20 |
| 50 - PC1 treated RS | <1.0 ± 0.00* | <1.0 ± 0.00* | 1.2 ± 0.35 | 1.5 ± 0.70 |

| | | | | |
|---|---|---|---|---|
| *Enumeration limit of 1.0 Log CFU/mL. | | | | |

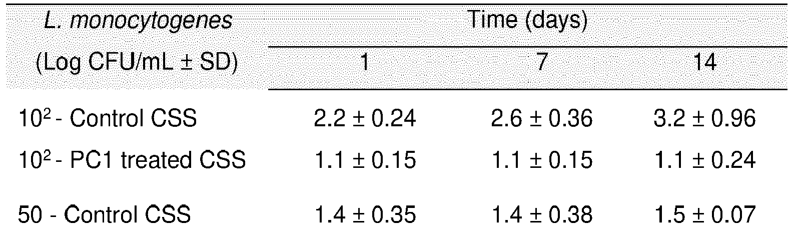

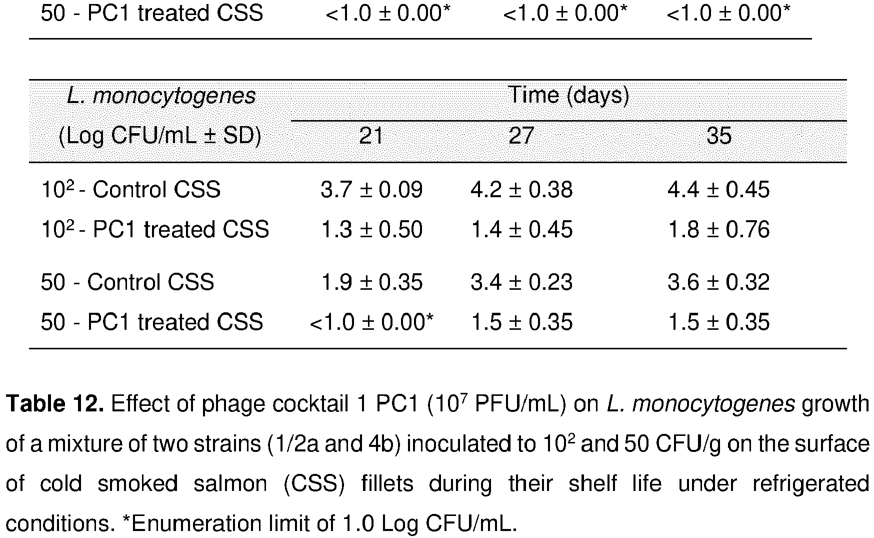

## Claims

1. A composition comprising the bacteriophage AZT451 deposited in the Collection Nationale de Cultures de Microorganismes of Institut Pasteur (CNCM) with deposit number CNCM I-5799.

2. Composition according to claim 1, further comprising at least one, two, three, four or five bacteriophages selected from the group consisting of bacteriophage AZT356 with deposit number CNCM I-5797, bacteriophage AZT154 with deposit number CNCM I-5796, bacteriophage AZT450 with deposit number CNCM I-5798, bacteriophage AZT153 with deposit number CNCM I-5795, and bacteriophage AZT150 with deposit number CNCM I-5794.

3. Composition according to claim 1 or 2, further comprising a carrier, preferably, a pharmaceutically and/or food acceptable carrier.

4. Composition according to claims 1 to 3, further comprising one or more bacteriophage-derived proteins, surface disinfectants, antibiotics, surfactants, enzymes, additives, preservatives, processing aids, protective cultures, further *Listeria* bacteriophages and/or further bacteriophages specific for bacterial contaminants other than *Listeria monocytogenes.*

5. Use of a composition according to any one of claims 1 to 4 for controlling, reducing and/or eliminating *Listeria* spp. contamination, or for preventing the growth or colonization of *Listeria* spp., in food- or feed-products, in pharmaceutical, veterinary or biotechnological products, in water systems, in fluids, in soils or iin non-food surfaces.

6. Use according to claim 5, wherein the food product is a "ready-to-eat" product, a dairy product, an unpasteurized food product, a vegetable, a meat, or a fish, or wherein the feed-product is fodder.

7. Use according to claim 5, wherein the water system is livestock drinking water, poultry chiller water, brines, hydro-coolers, brine coolers or chillers, cleaning water, baths and flumes for fruits and vegetables, and sewage.

8. Use according to claim 5, wherein the non-food surface is
- the surface of food, feed, pharmaceutical, veterinary or biotechnological processing equipment,
- the surface of food, feed, pharmaceutical, veterinary or biotechnological storage containers,
- the surface of pipes,
- soil,
- the surface of animal bedding,
- the surface of troughs, or
- the surface of an animal transportation container.

9. Use of a composition according to any one of claims 1 to 4 as an additive or preservative or processing aid in a food- or feed-product, in a beverage, or in pharmaceutical, veterinary or biotechnological products, preferably, the food product is a "ready-to-eat" product, a dairy product, an unpasteurized food product, a vegetable, a meat, or a fish, or preferably, the feed product is fodder.

10. Composition according to any one of claims 1 to 4, for use as a medicament, preferably, for use in the treatment of a subject infected with bacteria from *Listeria* ssp, preferably, *L. monocytogenes, L. innocua, L. ivanovii, L. grayi, L. seeligeri,* or L. *welshimeri.*

11. Composition according to claim 10, wherein the subject is a human or a non-human animal.

12. An *in vitro* method for detecting the presence of a bacterium of *Listeria* spp., preferably, *L. monocytogenes, L. innocua, L. ivanovii, L. grayi, L. seeligeri,* or *L*. *welshimeri,* comprising
(i) incubating an isolated sample with a composition according to any one of claims 1 to 4, and
(ii) detecting any change in said sample caused by the composition as an indication of the presence of *Listeria* spp., wherein the bacteriophage of the composition of the invention has been genetically modified to incorporate a marker gene.

13. Method according to claim 12, wherein the sample is from a subject, a food- or feed-product, a pharmaceutical, veterinary or biotechnological product, a water system, a fluid, a soil or a non-food surface.

14. Method according to claim 12 or 13, wherein said change in the sample is due to lysis by the composition or a detectable label or signal.

15. An isolated bacteriophage selected from the group consisting of bacteriophage AZT451 with deposit number CNCM I-5799, bacteriophage AZT356 with deposit number CNCM I-5797, bacteriophage AZT154 with deposit number CNCM I-5796, bacteriophage AZT450 with deposit number CNCM I-5798, bacteriophage AZT153 with deposit number CNCM I-5795, and bacteriophage AZT150 with deposit number CNCM I-5794.
